# DEMANDE DE BREVET EUROPEEN

(11) **EP 1 493 813 A1**
(43) Date de publication de la demande: **05.01.2005**
(21) Numéro de dépôt: 03447178.9
(22) Date de dépôt: 03.07.2003
(51) Int. Cl.: C12N 15/31, C07K 14/23, C12Q 1/68, G01N 33/50, C12N 15/11, C07K 16/12, A61K 38/16

(54) **Séquences génétiques flagellaires de Brucella destinées à un but thérapeutique et diagnostique**

(71) Demandeur: Facultés Universitaires Notre-Dame de la Paix, 5000 Namur (BE)
(72) Inventeur: Letesson, Jean-Jacques, 5170 Bois de Villers (BE); Lestrate, Pascal, 5170 Profondeville (BE); Tibor, Anne, 1350 Folx-les-Caves (BE); Fretin, David, 5100 Jambes (BE); Delrue, Rose May, 5340 Faux-les-Tombes (BE); Godfroid, Jacques, 1180 Bruxelles (BE)
(74) Mandataire: Van Malderen, Joelle

(57) **Abrégé**

La présente invention se rapporte à des séquences génétiques de protéines flagellaires de *Brucella*, leurs applications thérapeutiques et diagnostiques.

SEQUENCE LISTING

<110> FACULTES UNIVERSITAIRES NOTRE-DAME DE LA PAIX

<120> SEQUENCES GENETIQUES FLAGELLAIRES DE BRUCELLA DESTINEES A UN BUT
THERAPEUTIQUE ET DIAGNOSTIQUE

<130> P.FNDP.18/EP

<140> EP 03447178.9
<141> 2003-07-03

<160> 59

<170> PatentIn version 3.1

<210> 1
<211> 846
<212> DNA
<213> Brucella

<400> 1
atggctagca ttcttacaaa ctcgtcggct ctgaccgctc tccagacgct tgcttcgacc 60
aacaagtcgc tggaatcgac ccagaaccgt atctcgaccg gtcttcgtat ttctgaagct 120
tcggataatg cttcgtactg gtccatcgca acgtcgatga agtccgacaa caaggccaat 180
tcggctgttc aggacgctct cggcctcggc gccggcaagg tcgataccgc ttattccgcg 240
atcaacaaga tccgtgaatc ggttgacgac atcaagacca agcttgtttc ggctatgggt 300
gcgagcacag aagacaaggg caagatcgaa accgaaatca agtcgatcgt cgctaatatc 360
aactcggctc tcagcaatgc caactacgcg ggttccaacc tgctgaatgg cccgacgacc 420
gatctcaatg ttgtagcttc gtacaatcgt tccggtaacg ccgtcgcggt tgacaagatc 480
actgtcaagg ctactgacac tgatgcgaag accatggtta aagatatcgt agatgcgggt 540
ttcttcactt cggcctcgga cgatactgct attggtacgg ctctgaatac ggttgaaacc 600
gctcttgctt ctctcgcaac cggcgctgcg acgctcggtg cggccaagtc gcagatcgac 660
agccagaaga gtttcctgtc gggtcttcag gactccatcg aaaagggcgt cggtacgctc 720
gtcgatgccg acatgaacaa ggaatcggca cgtctgtcgg ctctccaggt tcagcagcag 780
cttggcgttc aggctctgtc gatcgccaac tcgtcgaacc agtcgatcct gtcgctgttc 840

cgcggc                                                              846

<210>  2
<211>  726
<212>  DNA
<213>  Brucella

<400>  2
atggcggtgg tgtggatgca gcagaatttc cagcaattga tcgaacagct caagggaacg      60
ctcggcaagc ttggtgcgcg aaagctcatc gcgctcggcc ttgtaggcgc ggctttgatg     120
ggtgcgatcc tctatacgag catctatctg ggtcgcccat cctacgagac gctctatgtc     180
ggcctttcgc gcgatgacgt gaaccgcatg ggccttgcgc tcggtgaagc tggcattccc     240
ttcgacgtga agtctgatgg atcctcgatc ctcgttccaa tcggcaaggc cgaaaacgcc     300
cgcatgtatc tggccgaaaa gggcctgccg acctcgaaca atgccggtta tgagctgttc     360
gacaatatgg ggtcgctggg gcttacgtcc tttatgcagg agattacccg tgtgcgcgcg     420
ctcgaaggag aaattgcccg taccattcag gccattcgcg gcgtgaaggc tgcgcgcgtt     480
cacatcgttc tggccgaaaa aggctcgttc cgccgcggcg accagaagcc gtctgcctcg     540
gtcgttattc gtgccgaagg cggattttcc gctgaatcgg cccagtccat ccgccagctg     600
gtcgccgctg ccgttccctc gctcgatgcg tcttccgtca cggtgctcga tacaaatggc     660
catctgctgg catcggctgg cgaaggggcc aatggtgcgg ctctcatgac cgcgtcgctt     720
gaacag                                                              726

<210>  3
<211>  999
<212>  DNA
<213>  Brucella

<400>  3
gtggacgaca gcatccgcaa ggcgcttgcg ccctatcttg gcctgggtca tttccagact      60
tccgttcagg ccgcgctcga caccgaccgc cgccagacca aggaaactac ctatgatccc     120
gaatcgcgcg tggagcgttc cgtccgtgtg gtgcgcgaaa gcggcgattc ccgcaacaac     180
cgcaatgata atgcgacggg tgtggagcag aacattccgc aggagcaaat ccagaaccgc     240
aacggcgaaa gctcgactga aaagacggac cgccgtgagg aactgaccaa ttatgaagtg     300
aacagcatga cggtttcaac cgtcagtgac ggctatagca tcaagcggct ttcaatcgcc     360
gtcgtcatcg atcaggcgcg cctgttgcag accgctggca ccacaccccc acccgccaat     420
ttcgtggacc agcagattac gaagattcgc gatctcgtcg ccaccgccgc tggtctcaat     480
accaatcgcg gcgatgtcat caacgtcacg gcggtcaatt tccttgactc tgccggtgcg     540

gatatggagc ctgtttccgc accgtggacc gatacgcttc ttcgtcagag tggaagctat 600

gccaatgcgc ttgccattct tgccgccgtc ggccttctga tctggtttgg cctgcgcccg 660

ctattgcgtg accagaatgt gaagcccgcg ggtacggaag tcgctatccg tgaggcgggt 720

gaggtcgcga cgccgaattt tatcggcggc gcggaaagcg tgggcgaagg tgttcaggcc 780

gtcattggcg gtcctgcggc ctatgccgat cagatgaaga cgagcctcag cgatctgcgc 840

cagcgtatgc gtatgccggc caagctcagg ctcgaacaga tgatcgaaat ggatgaagag 900

cgtgttgctg ccgttctcaa gcaatggatt cacgaaaccg cttcgggccg tgaggccgac 960

ccggcgaagg catcggccat gccggagctg aaggcggcc 999

<210> 4

<211> 678

<212> DNA

<213> Brucella

<400> 4
atgagcgcgc tttccatcag ccgatatctg actgacttct cgacccacca tattgacgat 60

aaggcggttg agatcattac cgcgccggtt ccgccaaaac cgctgacacc cgtagcgggc 120

gaggctgcgg aaaagcctgc cgcagtgggg caggagcagg cggaaatggc cgcagtgatc 180

gcgctggagg aagaaaaacg cgcggctttc gaggctggcc gtgaagatgg ccacagggaa 240

gcacaggtgc tttacgaggc tgaaaaggcg cgtctcctgc gtgaacatga agcggaaatc 300

gagacgctgc gtgccacatt ttcgcgcgaa caggctttgt tgctggcggg tagccttacc 360

gaagcactga gcgcgctgga acagagcctc tccggccaga tggctgaaat actgatgcca 420

cttctggccg cgaaaatgga gcatgaggcg gtagcggaat ttgcccgtcg catcgcggca 480

ctggcgctgg aaggtgaggc gccggaaatc tccggcccgg cccggcttct ggaagcattg 540

caggcccatg gggtgctttt gccctccggt tgccgctttt cagagacggc atcgaacgaa 600

ctttcctttt cctttggcga acggatgctc gaaacgcgca tcgcgccgct tcttgaagaa 660

ctgaaggctg cggtgaaa 678

<210> 5

<211> 1098

<212> DNA

<213> Brucella

<400> 5
atgaacattg agcccgaaac gaagcgcgag atcatcattg ttcgccgggg cgggcatgac 60

gaccatgagg cgcatcatgg cggggtgtgg aagattgcct atgccgactt catgacggcg 120

atgatggctt ttttcctggt catgtggctc atcaatgctg ccaatgagga gaccaaggcg 180

gcggtggcga gctatttcaa tcccgtcaag cttatagacc gccattccag cccgaagggc 240
gtgcaaaatg tggaccagcg cgatggcaag gttcgttttg aaagcgacca gcaggtggaa 300
agtagcacca aggtcgtcaa cgacaccact gtgcgcccgg caagcgacca ggaggagcgg 360
cagctcttcc gcgagcccta tgccatgctt gcggaattgg cgcatgaaac aggcgttctg 420
caaaatcagt cggcaaaggg cgatggcggc acggcgcaat ccggctccgc cagcggtgcg 480
gatggcggcg aggcctatcg ggatcctttc agcccggatt actggtcgag caaggcggtg 540
ggagacctga ccccgcctgc gccgcaaacc gtgccgcaaa ctgcaccctt gccgcaggcg 600
cagcccaaaa aggctgaaac gcaggaagaa ctgatcgccg atctgaagaa agccgcaaca 660
ggtgaaccgg ccccgaacgc gggcaaggcc gccaaacccg aaccgatgcc ggatgttacc 720
gtggttcccg tggaaggggg cgtgatgagc cagcttaccg acaaggttga ttatggcatg 780
ttcacgatcg gctccgccaa gccggatgcc cgtgtggtgc agatgctgga gcgcattgcg 840
caggtgattg cccgccagcc gggcgaggtc atcatcagcg gtcatacgga tgcgcgcccg 900
ttcaaaagcg cgacctatga caattggcgg ctatcctcgg cccgtgccca gatggcctat 960
tatatgctgg tgcgcggtgg ccttgatgaa aagcgcgtgt tgcgtgtcga gggctacgca 1020
gaccgccagc cgaagaaccc caacgatccg aatgccgcgg aaaaccgccg catcgatatt 1080
ttcatcaagt ccaagcca 1098

<210> 6
<211> 1290
<212> DNA
<213> Brucella


<400> 6
atgaagcggc ctgccttgcg ccccttgctc gtgttgatgg caagcctttc ttttgcggga 60
gtggcgggcg gaacggcctg tgcgcgggcg cagcctgaaa tgcggccctt gccggtgctt 120
gaaccctata agctcgtgcg ttcgttgcgc atgttgcagg atcaggtggt tgcgggcaag 180
ccggaagcgg tggtgatgct caacaagctt ctcggcttcg tcagcaccga tatgagccgg 240
gcgcagccgg aggtatggga caagccggaa aatatctatg ctgcaatcat ttacctattc 300
aatggcggca acccggaagc cgtgcgcaag gtgctggcta atctgaagac cgatactgtt 360
ccgcaggaac tggtgaaagg cgcgcttgcc tatgcttccg gccagaccat tgaggttgtg 420
aagcttttct ccgtgccgct ggcgccggag gttccggcgg aactcaaggc atccatcgtg 480
ctcgttaccg ccaatcagat gatggccttc gatccggcca cggcactttt gcggcttgac 540
caggtgcggc ttgaagcacc gggaaccctg ttcgaggagg cagcgatccg ccgctccttg 600
ccgattgcgg caaagcgtgg cgctgcagac aaggtcaggc ttctgtcgcg caattatctc 660
cagcggttcc ggcactcgcc ctatatgcgc gatttcatgg cacagtttgt cgataccacg 720
ctcaagctca gcgaccgtat cggcgatgag gaactggtaa agttgatcgg ctcggctgat 780

ccgctcttgc aatatacgct ctatcttcag attgcgcgcg gcgcgctggt ggatgggcag 840
acggaacgcg cacgcttcat gtcggcagag gcgcgcaagc tggccggtca tctgaacgcc 900
gatccgacgc gtgccaatct ctatgcggcg gcaagcgatg tggcgtcgga ttcggcaggc 960
agcgccttgc gggaactatc gcagatttcg cccgatcact tgcaggaacc ggatcgcaaa 1020
ttgctgaaag cggccgaaac ggttggggcg attgtcaccc gcgcacccga taccacatcg 1080
cgcgcaaaac ggagtgcgga cgagcgtgag gtgccacgca tggctgtggc ggatgcgccg 1140
caactgccag cccaacagac cctttccgcg ccggtggaag tggatttcaa tccagcccgc 1200
acgcgctccg aggcggactt gcagaagacg atggaaaatg cgcggcgcaa actggcagaa 1260
atcgatgcct tgttaggaaa gaccgttgaa 1290

<210> 7
<211> 1272
<212> DNA
<213> Brucella

<400> 7
atgagcgttg atatgttgct gacagcatct ggcaggctgg cctcccttgc gcgcaattcc 60
agcacgcagg ccagggtggc cggtgatgaa caggatggcg gctccgatcc ggctacgctc 120
ttcggtgcac ttcttgaaaa gccgcatggc aaggtcgatg ccagcggcaa ggatgaagcg 180
gaagcgaagt ccgatgaaaa ggaggctggc gacggcagcg ggcaaaagcc ggttgccgtt 240
ttcggcctgc cgcaaaacct gctttccctt gcttccgctt tgcccggcca gcagcatggt 300
gaagggggct tttctgtgga agaaaaggcg ggaaacccgg cgtgtgcagc cgtttcagaa 360
ccgggaatgg tggatgttgt tgatcccgct ggaattgata aggcggcagc agaatttgcg 420
ggcgatgatg aaaaacggcc cctccgtgag ttgccgcgcg tgaaagcggc acaaacgaat 480
ggcgatctgg ctatcagggc agggcaggcc tccggtgagg ctgctttgga tatatctggg 540
gatatatcgg gcaacaagct ggcagaaggt gctgcggctg atgttgccgg tattaaggcg 600
cccaaaccgg caaccgatca tgagccggca aaaggcatgg acgtgcgtgc cgacctcctg 660
ccgcaacagg ctgccgcaaa aagtacggtg ccagcagaag ccaaagctgc ggctggcatg 720
ccggaggcag cacggattgc ggatatagag gttgtttcgg agcgcagttt cggcacggtc 780
aagaccttgc agatcaggct cgatccggtg gagcttggcg cggtgacagc gcgcatccgc 840
gtcgcgggcg acagcgtgga agtgcatctg gtggcggaca aatcccatgc cgccgaaatg 900
ctcgctgccg accgttccat gattgaaaag gcgctgaaag tggccggtgt cggcgacgat 960
acgaagattt cagtcacggt ggcagaccgc aatgcgcagg gcgctgcgca acatgtggcg 1020
gcggcgcaga atgcgggcca gcagcaggca agcgcgcaac agcaggggca ccaactggcc 1080
ttcaatatgc agcagcaggg ctcgcaaggc cgtgggggcg aggctcaggc gcagttcatg 1140
agcggtagat cgggcggaga gggtggccgg aatggcggat cgggtcaggc gggacgtgaa 1200

catgcgaata gtcaggcgaa gcccgaaaat ggccgtggcg ctcctcatat cggtggtcgc 1260
ggtctggttg tc 1272

<210> 8
<211> 516
<212> DNA
<213> Brucella

<400> 8
ttggcgcagg cggaaaatat ctgcgagcgg gaaatgcacc gggcatcggc ccgttacggc 60
gttccgctca gtattctcta tgccgtgggc ctgaccgaaa caggccgcaa gaattcgctg 120
caaccctatg cgatgaatat tgagggaaag gccgaattcc cgccctcgca atcggccgca 180
atcaggcgtt ttggcgaagt gcgggccgaa ggcgcgaagc tgattgatct tggttgtatg 240
cagatcaact atcattacca cagtcaggcc tttccctccg tggcggcaat gctaatgccg 300
agcctgaatg tggactatgc ggcgcgcttt ttgaagcggc ttcgagaacg ggaaggcaat 360
tggaccatgg ctgtcgcgcg ttatcatgcc gggccgaata acgatcccgc gcagaagcgt 420
tatgtctgcc gcgtgatggc gaatatggtg gcaacgggct ttggaaactg gacgcctcag 480
gcacgaagct tctgtttcag ccgcggcggg cttttg 516

<210> 9
<211> 681
<212> DNA
<213> Brucella

<400> 9
atgattgttg tcgttgacga cagagacatg gtgactgaag gatattcttc ctggttcggt 60
cgcgaaggga tcacgacaac aggtttcaca cctaccgatt tcgatgaatg ggtcgagagt 120
gttcctgaac aggatattat ggcgattgaa gccttcctga ttggtgaatg cgccgaccag 180
catcgcctcc cggcccgcat tcgtgaacgc tgcaaggcgc ctgttatcgc cgtcaacgac 240
cgtccttcgc ttgagcatac acttgagctt tttcaatccg gggttgacga tgtggtgcgc 300
aagcccgttc atgtccgcga aattctcgcg cgcatcaatg cgatccgccg ccgtgcgggc 360
gcatcggcaa cctctggcgc agatggcacg caactggggc cgatccgtgt tttctccgat 420
ggccgcgatc cgcagatcaa cggtatcgat ttccgctccc cgcgccgtga gcgccgcatt 480
ctggaatatc tgattgccaa tcgcggccgt cgtctcaaca aggtgcagat ttttagtgcg 540
atttacggca ttttcgacag tgaagtcgaa gaaaatgtcg ttgaaagcca tatcagcaaa 600
ttgcgcaaga aattgcgcgg acagcttggg ttcgatccta tcgattccaa gcgtttcctg 660
ggctactgca tcaatatcga g 681

<210> 10

<211> 1188

<212> DNA

<213> Brucella

<400> 10
atgagcctct acggtatgat gcggaccggt gtttcgggaa tgaatgctca ggcgaaccgt 60

ttgtcgacag ttgcggataa tatcgcaaat gcaagcacgg tcggttacaa gcgcgcggaa 120

acgcaattct cctcgcttgt cctgcccagc actgccggac aatataattc cggcagcgta 180

ctgaccgatg tgcgctatgg catttccgat cagggcggta ttcgttccac gtcttccacg 240

accgatcttg ccattgatgg caatggctat ttcgtggtgc aaggccccgg cggctccacc 300

tatctgacac gggccggttc tttcgtgccg gacaagaacg gcgatctggt caattccgcc 360

ggttattatc tgctgggtgc gggcgcggac gaggcggcgg gtgggctgac cgtcgcgggg 420

cttaatatcg tcaatgtcaa cgcggccgct ttgccggcag agggaagcac ggcgggcgat 480

ttcaccgtca acctgccttc caccgatcag gcgcctgcgg ctggcggata taaccacaaa 540

acctcgctca tttcctataa tgacaagggt gagaagatca cgctcgacgt ctatttcacc 600

aagacgggtg cggatgaatg gaatgtttcc gtgaagaacg ctgccgatgg cgtggagatc 660

ggcacgacgg tgctgaactt cgatcccaca acgggcgatc tcgtttccgg tggcaatgtc 720

gcggtcaatc tcggtgccta tggtggccag accctcaatc tcaatctggg cggttccacg 780

cagcgtgcgg gcgattacac gatttcacag gcggtcatca acggtcaggc gccttcatcc 840

atcaagggtg tggatgtcgg taatgacggt gcggtggtcg cggtctatga aaatggcacg 900

cagaaggttc tctaccgtat tccgcttgcc aatgtagcaa gcccggaccg catgacggtg 960

gtgagcggca acatattcct gcccagtgcg gaatccggcg atgtccgcct tggcttcccg 1020

cagggcgatg gcatgggcaa gatcatgtcc ggcacgctgg aggaatccaa tgccgatata 1080

gcgcaggagc tgaccgacat gatcgaggcg cagcgcagct atacggccaa ttccaaggtt 1140

ttccagacgg gtttcgagct gatggacgtg ctggttaatc tgaagaga 1188

<210> 11

<211> 1452

<212> DNA

<213> Brucella

<400> 11
atgtcactta gttctgctct tctgacggcc aaaagttcac ttgcggcaac gtccaagcag 60

acgtccgtgg tttcccgcaa tatttcaggg gcgaaagatg cggattattc ccggcgcacg 120

gcctcccttg tatcgggccc ctatggctcc ctttatgtgg ggatcagccg gtcggcggat 180

gaagcgatgt tcaatcgcta tatccagtcg aacagcgccg cttccgcatc gtccactctc 240
gcagacggtc tggaccgcct ttccgcgctt tattcggcgg ataattattc cggctcccct 300
tccggcctta tcggcgatct gcgcgacgca ctccagacct atgtcgcttc gccttccaac 360
agcgcgcttg gcgatagcgt ggtctcggtt gcgcagtcgc ttgccaatgc gttgaatgac 420
tgtacgcggc aggtgcagtc gctgcgcaac gatgccgacc gggaaattgc ggattccgtc 480
gccaatatca acgatcttct ggcaaaattc gagaaggcca atcagaacgt ggttggtggc 540
acccgcatgg ggcgggacgt gtcggattat ctcgatcagc gcgatgcatt gctgaaacag 600
ctttcaggcg agatcggcat cacaaccatg atgcgcggtg acaacgacat ggttattttc 660
gccgaaaacg gcgtcaccct gtttgagacg acggcacgca aggtcacttt cgagcaatcc 720
gccgttctga cgccgggcgt ggcgggcaag gcggtgacgg tcgatggcgt gccgctcagc 780
catgatacgt tcgaccagcc ttttggtacg ggccgcttga gcgggctttt gcaattgcgc 840
gaccagatcg caccgcaata ccagatgcag cttgatgaaa tcgcacgcgg gctggtgacg 900
gtgtttgccg aaagcgacca gacgggttct agcccggacc agaccgggct tttcagctgg 960
agcggctcgc ccgccatacc gggtgcaggc ctttctgccg gtatcgccgg aacgatcgag 1020
gtgtccgtgc cgttcattgc ttctgaaggc ggcagcgcgc ttcttctgcg cgacggcggg 1080
gccaatggcg cgaattataa gtataatgtg caaggcgctg ccgggtttag tgatcgcctg 1140
cgcgcgctga acgaagcctt ttccgaaccc atggtttttg atgcagcggc ggggatttcc 1200
tccagttcaa gcctgatcgg ctacagcgcc tcgtctctgg gctggcttga aggcaagcgc 1260
cagaaagcca atagcgaatt tacctataat gggacggtcg ccagccaggc cgattttgct 1320
ctttccaacg ccactggcgt cgatatcgac actgaaatgg cgctgcttct cgatcttgaa 1380
cattcctatc aggcatcaag ccgggtgctg acgacggtta gcgcaatgct tgatgatctt 1440
cttaacgcgg tg 1452

<210> 12
<211> 1044
<212> DNA
<213> Brucella

<400> 12
atgaaagcgc aatcgatttc cacctatggc gcaacatcag ccctgcgggc ccttgtcgcc 60
aagaacaagg ctgaaatggt gaaggcacag caggaagcaa cgaccggcac tgtgttcgat 120
gtcgggctgt cgctgggttc tcggacgggg cagacggttt cgctgcgcaa ggaatatgac 180
cggctgagcg ttttgaccga catgaacaag ctggtccagc agcgcatgac cgcaacgcag 240
accgcagccg gcaagatcat cgagaatacg cagaacttcc ttggtgatct ggcaggggcc 300
aataattccg gtgaaacggc gaagaccgtc gctaaatcgg cgcgctcgat gctggattcc 360
gttaccggtc ttctcaacac cagcttcaat ggcgaatata ttttcgccgg tgtgaatacc 420

gatgtgaagc ccatctccga ttatgcggat ggcagcaccg cgcaaaatgc cgtgcgccag 480
gcctttcagg atcatttcgg tttcgccatg gatgatccgc aggtggcgaa tatcagcggc 540
gacgagatga aagcctttct ggaaggtgat ttcgcagaac agttcaacga tgccaattgg 600
gcggccaact ggtcggatgc ttccgacacg cgcatcaaga gccggatttc gcccactgaa 660
acagcggata cctccatttc cgccaatgcg gatggctttc gcaagacggt catgtcggcg 720
gttatggtga cggaattcgc agatatcggc ctcaacgctt cggctttcga tgccctgaca 780
acacaggcct tgcagatcac gacacaggcg gtcaccgaaa cgacatcgga gcagacgacg 840
ctcggccttg cccagtctcg caccgaggcc gcgacgacgc gcattgccgc gcagcagaag 900
atcctgaacc agtccgtgct caatcttgaa gaggtcgatc cctatgacgc cgcaacgcgt 960
gtgaacgcgc tcaagacgca gatcgagaca tcttattcgc tgacggttca actacagaat 1020
atgagcctcc tcaactatct gagg 1044

<210> 13
<211> 342
<212> DNA
<213> Brucella

<400> 13
atgtatcagt tgcgctacga agatgtcatg aatgacgata tggcgagcgc caaggaacgc 60
gagcggatgc ttttcgaccg caccattgcg atgctcgaag cggcgagggc aaatggagcc 120
gaatcgcgcg aggggatcga tgctgcctat ttcacttcaa aactctggac gacgatcatc 180
gatgatctgg gatcggagga aaacgccctg ccgaaagaat tgcgggcggc gatcatttcc 240
atcggcatct tcgtgctgaa ggagattgaa cgtatccgcc agggtgagag caatgattat 300
gcaacgctga tcgagatcac ccagtcgatt cgggacgggc tt 342

<210> 14
<211> 456
<212> DNA
<213> Brucella

<400> 14
atggcggcga acagcaaaac ggcaatccgg ctgtcgctga gggcagggga gcggattttc 60
atcaatggcg cggttctgag ggccgaccgc aaggtctcgc tcgaacttct gaacgatgcg 120
acattccttc tggaaaacca tgtgctccag cccgaagaca ccacgacgcc gctgcgccag 180
ctttatttcg cggcacagat gatgctcatc gaacctgcca tgcgcgagca ggcgggcgct 240
actttcgccc agatgctgaa aggcatgttc gccacgttca aggatgcgga aatcctgaac 300
gcgctgaaac tagtggatga actggtgcat aacgggcgcg ttttcgaggc gctcaaaacc 360

attcgcgcgc aatatccgcg cgaggctgaa cttatgggtg cgcagcccgt ggtatggcct 420
gtgacgaaat ccgggaaatc tgcgggggca aatcca 456

<210> 15
<211> 411
<212> DNA
<213> Brucella

<400> 15
atgacgacga catctccggt tggctcgaac acgacgaaca gtgccagtac ggccagcaac 60
agcacttccg ccgccaacaa ggcgagcgtc gattatgact cgtttctgaa acttctggtc 120
acccagatgc agaatcagga tccgacacaa ccgatggacc caacgcaata tgtgtcgcag 180
cttgcaactt tctccaatgt cgagcaatcg gtacagatga acagcaagct ggaaacgctg 240
attgccaata cctcgctcac acaggccgag ggctggattg ggcgcacgct caccaatgcc 300
gatggcagca tttcgggcgt cgtgaagtcc gtcaccatcc agtccagcgg tatgctggcc 360
gaactggaag acggcaagac actgacgatc ggcgaaggtg tcaggatcag c 411

<210> 16
<211> 264
<212> DNA
<213> Brucella

<400> 16
gtgaacgagg ctgatgcgct tgatatcgtc aattcggcga tctggaccgt gctgaccgca 60
agcgggccgg cggtgcttgc cgccatgctt gcgggcattg ggattgcgct gtttcaggcg 120
ctgacgcaaa ttcaggaaat gaccctgacc ttcgtgccga aaatcatcgt cattttcgtc 180
gtcctggcgc tgacggcacc ttttgtcggt gcgcagatca atgctttcac gctgcttgcc 240
tattcccgca tcgaaaaggg tttt 264

<210> 17
<211> 297
<212> DNA
<213> Brucella

<400> 17
gtgcagcagg ctgcggcaaa acctttcata aaaggcaaat ttctgaccgg cagcgatatc 60
gggcttgccg tcggcatcat tatcatcctg accgtgcttt tcctgccggt tcccgctgtt 120
gtgctggata tcgggctcgc cttttccatc gccttttccg tgctgatcct gatggttgcg 180

ctatggatcc agcgcccgct cgatttttcg gcctttccga ccgttctgct gattgcaacc 240
atgatgcgat tgtcgctcaa tattgcgacg acgcgcgtga ttctcaccca cggcaat 297

<210> 18
<211> 1767
<212> DNA
<213> Brucella

<400> 18
gtgatccacg gcttttcgca gttcgtgatg ggcggcgatt tcgttatcgg cctggtcgtc 60
ttcgccattc tcatcatcgt caattttctg gtcatcacca agggtgcgac ccgtatcgcg 120
gaagtgggcg cgcgcttcac gctcgatgct attcccggca agcagatggc catcgatgcc 180
gatctttcat cgggcctgat cgatgaaaag gaggcgcagc gccgccgccg tgaactggag 240
gaggaaagct ctttctttgg ttccatggat ggcgcgtcga aattcgtacg cggcgatgcg 300
attgccggtc tcataatcac ggccgtcaat atttttggcg gcatcgtcat tggtgcgacc 360
cgccatggga tggatatttc gcaggccgcc gatgttttca ccaaactgtc ggtgggcgat 420
ggtctggtaa cacagattcc ggctctgatc gtttcgctcg ctgccgggct tctcgtgtcg 480
aaaggcggca cgcgcggctc tgccgatcag gcgattttcg gccagcttgg cgcttatccg 540
aaggcgcttt tgattgcggc gctcctgctt ttcattctgg gcgtcatgcc gggccttccg 600
gcctttccgt tcttcctgct cggcggcgca atggcgttcg tcggcattgc cgtgccgcgc 660
cgccaggcgc gccagcgcga ggccgatgcc gccgaagcgg gcaagaagca gcgcgaggcg 720
gaagagcagg aacgcaattc ggtcaaggct tcgctggaaa ccaaccagat cgagctttgc 780
ctgggcaagc agctttctgc ccggctgatc gcctcgcaag aggaactggc gcaccgcgtc 840
aacaagatgc gccgcaaatt tgcgcaggaa tacggtttcg tcatccccga aatcaaggtc 900
acggatgata tcgcgctacc gccgaaaagc tatcgcatta aaattcacgg cacagcggtt 960
gcaagccatg aattgcgtgt gggcgaaatt ctggtcgtgc tgggcgaacg tccggttcca 1020
tccgtgccgg gtgaggaagt gcgtgagcct gctttcggca tgcgtgctta ttcggtgccg 1080
gaaaccttca ctgccgattt gcgccgcgaa ggttacatga cggtggataa tctgtcggtc 1140
ctgctgacgc atttgagcga gatcgtccgt aacaatctgg cgcagcttct ttcctataag 1200
gatatgcgca tcctgcttga ccggctcggg ccggaatatc gcaaattgct ggaagatatc 1260
tgccccgcac atatttccta ttccggtttg caggcggtgc tgaagcttct tttggcagag 1320
cgcatttcca tccgcaatct gcatctgata ctcgaagcga ttgccgaaat tgcgccgctc 1380
gttcgccgcc cggaaatgat tgtcgagcat gtgcggatgc gcatggccca gcagatttgc 1440
ggtgaccttt ccgataatgg cgtgctcaat gtcctgcgcc ttggcaaccg ctgggatctg 1500
gtcttccacc aaagcctcaa acgcgacgcc aagggcgaaa tcgtggaatt cgatatcgat 1560
ccgcgccttc tggaacagtt cggcacggag gcctccgccg ccattcgcaa gcatttcgat 1620

aatggcgagc gattcgtact ggtctcctcg ccggaggcgc ggccctatat tcgcatgatt 1680
atcgaacgcc tgttcgccac gctgcccgtg ctttcgcatg tcgaaatcgc ccgtggcgtg 1740
gaggtgaaat cgctcggcgc gatttcc 1767

<210> 19
<211> 765
<212> DNA
<213> Brucella

<400> 19
ttgcctgtca cgcaactgcc gctcaacgaa atcatcctgg ccgccgtgct ggcgttttgc 60
cgcattggcg catgtctcat gctgatgccg ggaatttcaa gcgcgcgcat tccgttgcag 120
gttcgcctgt tcattgcgct cgcctgctca tttgccgtgc tgctgcttgt tcttcagcgc 180
atcgtgacgg cagtggatgc gggacagcct tggacgctgt ttcgcctgat ggtgggtgaa 240
atgttcatcg gtgcgctgat cggcattctg gcgcatattt atttctgggc gctgcaattc 300
atggcgaaca tgatggcgat ggcggttggc tattccggtt cgcctgccga ttcgattacg 360
gaaaacgagc cgcaggcaac ccttgcgacc atcgtgactt tcagcgcgct gtttctgttt 420
ttcgtgaccg atatgcatct ggaaatattc cgggcgctgc tcaattctta cgtggcaatc 480
cccgtcgatg gccggtttcg gccggatgcg gcgatgatcg attttaccga tgcgctttca 540
gcggcctttc tggcgacttt gcgtatagcc gcgcccttta tcgtctttgc cattctggtg 600
aatttcgcaa tcggcctcgt caacaagctg acacctgcga tcccggtcta tttcatctcc 660
atgccttttg tcctgggggg agggctgctg gtggtctatt tcatcctgcc ggagcttctg 720
cgctttttca cgcatgaaac cgcaacccag ttgcgcgaac ttttc 765

<210> 20
<211> 405
<212> DNA
<213> Brucella

<400> 20
atgacgcagg ctggaacacg caatctgagg aaactggtgg aactgcaaaa gcttggctgt 60
gcgcggcacg aagcagcact tgccatcgca aatgcgcgaa agtcggcgct tgatgaggag 120
cgcgcggcgc tgattgccat gcaggaccgg cgctatgacg cgaatgcgct cgatattgat 180
ccctcgctcg tcatccggtg gctggagacc aatgcggttg aaatgcaaca ggtcgaatca 240
cggctcgaat tggcgcgaaa agcacttttg aaagagcaac ggcgcgtgga acttctgcaa 300
gaccggctga acgatgcgca ggctgaccgt gagcggcgcg agcttgccag cctgatcgaa 360
gaatttgtca gccgcaaaac atcggatgag tcgcaaaagc ggagc 405

<210> 21
<211> 588
<212> DNA
<213> Brucella

<400> 21
atggcgatca acccgccgtc agatctggtg atggacgtgg cgcgcgccgc cgatccacag 60
gcctatcgca tggcggcgga gcggctgaat gcggcatccg gcgttgctgc gccgatgggc 120
ggttctgcgg caacagggct gacgcaggat aattttggaa gcttttccga aaatctcgcg 180
gctgaggtca gcgtacggcc ggatgcgcaa agcgcggcca gcccagccta tcgcaagttt 240
gaggctttca tgctgcaatc ctttgtgcag tcgatgttca ccagcgacac gactgcgact 300
tttggcaagg gcattgccgg ggaatactgg aagtccatga tggcggaagc catggcgaac 360
aagatggcag atggcggcgg tgtcggcatt gccaggttac ttgaagaaca ggcggcgcga 420
aacaggcggg cagaggcgcc ggcaaccctg gccctgggag atgtaatcga tactctggat 480
gtgaatgcgg gtgaaaaggc ggtttcaaag gacattattc atggccttga acgcaagctt 540
atccaaaagc aactcggcaa caataacagc accgaccgtg cgcacggc 588

<210> 22
<211> 342
<212> DNA
<213> Brucella

<400> 22
gtgcgccgct tcccgtcttt ccgtccatct gagcaggttg cccccgcatc cgaggtggat 60
aaaaccatga ccggctccgt catgtcgaat ggcaaggggc ttccgggcgc aggcaagggg 120
gagggcgaga ttggcgacgg cagggggcag cctttcccga agccggtctt tgcgctgcgt 180
gacgtggtgg gcggcatggc gatgatcgaa gatgcgtcgg gttattggtt cgttgagaaa 240
ggctcgctcc tgccggatgg cagccgcctc gtttccatcg gccgcggcgc gggcacctgg 300
cagcttacca cctcgtccgg cgatatcatc gatatgcggc gg 342

<210> 23
<211> 1140
<212> DNA
<213> Brucella

<400> 23
ttgcaggtgc tggtgaaacc gttcgatgac cggatcatgc cttcgctggg ggcagcggtt 60

ttcgaggaag gcccgttgcg catccgcccg gcgccggaat ggcggggccg ggtcatcaat 120
gcgcttggaa atgccattga cggaaagggc gcgctgaaac cgggcacccg ccccatggcg 180
gcagaaagcc ttgctccggc ggctttgcgc cgtgcgcggg tggatcgcgg cttgcgtacc 240
ggcgttaatg tgatcgatat tttcacgccg ctctgttttg gccagcgcac cggcattttc 300
gcaggctccg gtgtcggcaa atcaacgctt ctcgccatga tgacgcgcgc tgccgatttc 360
gatacggtcg tgctggcgct gacgggcgag cgcgggcgcg aagtgcgcga aatgctggaa 420
gaaaccatgg ccgggcattt gggcaagacc ataactgtgg tggcgaccgg cgatgaaagc 480
ccgatgatgc gccgccttgc gcccaatacg gcgacggcga ttgccgaata tttccgcgat 540
cttggccaga atgttctgtt gatcgtcgat tccgtcacac gctttgccca tgcggcacgc 600
gaagtggcca ttgctgccga ggagccgccg gttgcacgcg gttatccgcc aagtgttttc 660
agccagttgc cccgccttct ggagcgtgcc ggtcccggca gtgccgaagc gggtggcagc 720
atcaccggta tttattccgt gctggtggat ggtgacgatc ataacgatcc ggtttccgat 780
actattcgcg gaacgctgga cggccacatt gtgctcgacc gcgccattgc cgcgcagggg 840
cgctttccgg cggtggatat tccggcttcc gtttcgcgcc ttgccaaaca caactggacg 900
ccggaacagc gtaagctggt gatgcaattg cgcagcatgg ttgcccggtt cgaggaaacg 960
cgcgacctgc gcgccattgg cgcttatcag aaaggccatg acgggcttct ggaccaggcg 1020
gtcgattttg tgccgcgtat ttatgaagct ttacagcaat caccggaaac cggcctatcg 1080
gacgatccct ataacgacct tgccgcagca ttgcgcggtg acagacaggc gcaaaacgca 1140

<210> 24
<211> 246
<212> DNA
<213> Brucella

<400> 24
atgacgcctg atctgccact tacccgcctt gctgcttttg ttcgccagca aacaatagcg 60
ccagagccgc tcaccggtat cgggggcacg gtgagtgacg tttcgcgttc gtctattgca 120
gtgcgtggcc tttcgcgcga tgcgcggctt ggcgatacgg tggcaatccg cgctggcgta 180
gggggcgccg agccttgccg aaatcatccg tgttgccgat ttgcaggtgc tggtgaaacc 240
gttcga 246

<210> 25
<211> 519
<212> DNA
<213> Brucella

<400> 25

atgcagaaca attcgatcta tgtcgggctt tcctcgctca tcacgctgga acggcggatg 60

gatgcgattg ctcataatgt agccaatgcc tccacggtcg gctttcgcgg cgagggtacg 120

aagtttgaca ccatcgtttc cgacaaggcc agcgacgatg tgagctttgc cacagcgggc 180

aagagctata ttcgcacgga agcaggcccg ctgatcaaga ccgacaatcc gctggatgtg 240

gccgtgaagg gcgatgcatg gatgtcgttc tccaccccgc aagggcaggt ctattcacgc 300

gatgggcgca tgaagatgct gtccacaggc gaccctgttt ccgtcaccgg caatgcgatc 360

ctcgatgtcg gcggtgcgcc cattgtgctc aatccgggcg gtggtgcgcc caggatatcg 420

agcgacggcg cgatctacca gaatggtgta caggttgggg cgatcggcct ttacaccatc 480

ccggccgatg ccgatctcgc ctatgcggca cttccggcg 519

<210> 26

<211> 786

<212> DNA

<213> Brucella

<400> 26
atggtcgata cgatgacgag ctatcgcctg atcgcgtcga atatgacgcg gtcattgcag 60

cgcgtttcaa aagagccgct ggtggagcgc gaaacggcct attacaagga aaatatcggc 120

aatgtgaaat cggttgatga tttcatggcc gatacgcggc tcttcaatta cgccatgaag 180

gcgttcggcc ttgaagacat ggcctatgcc aaggctttcg tgcgcaagat cctgactgaa 240

ggcgtcaccg atgaagacgc gatggccaat aagctgaccg acaaacgcta caaggaattt 300

gccaccgtct tcgattttgc aggcaaaggc gcgcaggcca cgcagagtac ggcggcgcag 360

cagggtgtga ttgacaaata tgtccgccag acgctggaaa aggaagcggg cagccagaat 420

gagggcgtcc ggcttgcgct ctatttcgag cgcaaggcgc cctcgctcac caatgcctat 480

gaaattcttg gcgacaaggc attgctgacc atggtgcaga cgacctttgg ctggccgtcc 540

accatgtcca atgcagatat cgacaagcag gcgaaaatga tcgccgacaa aatcgatttt 600

tccaagatgt ccgatcccga ctatgtatcg aagttcattt cgcgcttcac ggccatgcat 660

gaaatgaaca atccgaccgc gagcagtccg gcctccatcg catcgtttct tctgggcggc 720

acctcatcca tcggcatttc gatgaacatc ctcgcaaccc tgcaaaactt caagccgggt 780

gggcgc 786

<210> 27

<211> 969

<212> DNA

<213> Brucella

<400> 27

ttgtccgaag ctggtttccg ccagcaccgg cggcaagctt cgcgcaagcc gcagttcata 60
aggtgcgatg aaagaacttg tatgcggggg tttctggctt tgggcattat cgtcggtctc 120
gtcatcacgc tgggctgtat gctgggcggc ttcattgcca tgggcgggca tgtcagtgtg 180
ctgatccagc cgtgggaatt catcatcatt ctgggcgccg cactcggcac ctttttcgtg 240
gccaatccct tttcgctcgt gaaagacacg ggccgcgcct gcatggaagc cttcacggac 300
gccgtgccga agcagcgcga ttatctcgat gttctgggcg tactctacag cctgatgcgc 360
gagctgcgcg ccaaatcgcg caacgaggtg gaagtccata tagacaatcc gaaggaatcc 420
ccgattttcc tcgcttatcc aagcgtgctg aaaaaggaag acctcaccaa tttcatctgc 480
gactattgcc gtctcatcat tgtcggcaat gtgcgctcct atgaaataga agccctgatg 540
gatgaggaaa tccgcaccat cgcgcgcgac aagctgaaac cctatcaggc catgataacg 600
atctcggaag ccctgcccgc gctcggcatc gtcgccgccg ttctaagcgt catcaaggcc 660
atgggcgcgc tcgaccagtc gccggaagtg ctgggccatc tcatcggcgc cgcactcgtc 720
ggcacctttg ccggcatctt cttctcctat ggcgtgatcg gccccatcgc caacaagatc 780
aaatcgaccc gcgagaagaa caaccgcctc tatatcgtcg tgaaacagac gcttcttgcc 840
tatatgaacg gctccctgcc gcagatcgcc gtggaatatg gccgcaagac catttccgcc 900
tatgagcgcc cgaccatcga cgtggtggag caggaaacca tggcaagcgt gccaacacag 960
caggccgcc 969

<210> 28
<211> 948
<212> DNA
<213> Brucella

<400> 28
atggatgacg ccacaacgct ggagcataac cgtaaaaacg atgccctcgc ggaaaatctt 60
ctgcgtgcgg cagggctttc gggcgacgac ctgaaatcgc tggcgcatgt gttcaaggac 120
gcctcctcgc atttctgcgc ccggctgaag cagggttccg cggcagcctt cgacgctgaa 180
accggctcgg tggaaagcac cgacaaaacg gcgttcaacg atattttcga caaggctgcg 240
atcataacgc ctctgaaggc ggaacgctgg ggctgctcgc tttatcttgc gctcgacgcg 300
gttctcgtct tctcggtgat tgaggccatg ttcggcgcac aaggcaatct cggccctgtc 360
gatgccgatc accccttcgg catggtggag cagaaaatcg caaatcttct ggcaggccat 420
cttgcgggcg cgctggaccg ggttttcaat accagctcca gccagccgct atttgcgccc 480
ggcgattgta tcgatacggc ggatttcgac cgtgaaaact tcgagctttc ccggcttttc 540
acctgccgca tcgcggtgac ggcggcaggc aagaccggcc agatgcatct gcttttgccg 600
cgcagcaccc acaagccgat gcaggatgca gttgccgcct ttcttcgccg cccgatggat 660
caggccgacc cggcctgggc caaaaagctt cgccaggagg taagccgtgc gcgcatcgaa 720

ctggaagcct tcatgcagca gggatcgatg tcgctcgatg cgctctcccg gctcgaaatc 780
ggacaggtgc tgaaactgcc cgtcgacgcc atggaacagg tccggcttcg cgccggaaac 840
cagcagcttt tcaaatgcac gcttggcaaa tccggcattc atttcacagt caaggtcggc 900
gacccggtca atcaggaaga ggatctcatc gatgagcttg ttgctggc 948

<210> 29
<211> 459
<212> DNA
<213> Brucella

<400> 29
atgagcttgt tgctggctaa cgtgctttcc acattgatgg ctcttgcctc gcttgcggct 60
ttgatcgtgg tgggccgcaa ggccaatgaa accatcaggc tcggcaaggt gctggcgctg 120
cgcgtcgccg ccgcgtcaaa cggtctggag cgcgccgtaa aggcgatccg caacgaacgt 180
accgacctta ccgacgccag cgccaagctt gacgcccgcc ttgccgaaac gcagcgcgtg 240
cgccgtgaaa tggacgaggc gatcgaggaa ttgaaccggc tgcgcaaggc gctctcgcgc 300
gatatgcgcc aggcgtgcat cttggccgaa acgccgccac cccggcagga gaaaaccccg 360
tccgccgcag aacaaccgct ccccgcagac aagaacgcgc tgcccgtttt cgtgcgccgc 420
gcggtcaaga aggcaaccgt tgaactggcg ggacaggca 459

<210> 30
<211> 318
<212> DNA
<213> Brucella

<400> 30
atgcagcagg aactgaacga agccattgag gaactgcgcg aggaaaagcc acgcagcgcg 60
caaaaaagtg cttccaagcc caatcttgat ctcatcatgg gcattccggt ggatgtgcag 120
gtggttctcg gcggcacgac catgccggtt tccagcctga tgaagctggg acgcggtgcc 180
gtcatcacgc tcgacaagca gatcggcgat ccggtcgata tcgtggtcaa cgggcgggtg 240
atcgcgcgcg gtgaagtgat cgttctggaa gacgattcct cgcggttcgg cgtcagcctc 300
accgaaatca tcggcaag 318

<210> 31
<211> 696
<212> DNA
<213> Brucella

<400> 31
atgagcgaca ccgagcagca catcctcgcc gaaaccggcg ccgccgggcc gggcaacagt 60

attgtcgaca ccctgaccgg cccgcaaaag gcagccgcga tactggtcgc catcgggcgt 120

ccggcggcgg cacggcttct gaaacatttc aatgccgagg atttgcgcaa gcttgccggt 180

catgcgcgca cgctggagcc gatttcgccg ctcgattttg aacagctcgt caagcagttt 240

gaggatttat tcgccgaagg cgcacccgtt tcgaaagccg cccagcgctt tgaggggctg 300

ttgcgcgata cgctgccgca ggaagaagtg gacgcggttc tcgaagaccg tgtgcagccg 360

caactggtgc aggaatcggt atggatacag gttgcccgcc tgccgatgga aagcctgcaa 420

gcctatctcg gcaaccagca tccgcagatc atcgcctata tcgtatcgcg gcttccctcc 480

gaccttgccg cgcgcctgtt cgtggccctg ccgccgcaat tgcgcaatgc cgtggtgcag 540

cgatgccggt ccgctcaagg cgcatcacgg ccagattgcc aatatcttca accagctcga 600

ccgaagcgag atggaagaac tgatggcaag cctgcaagac ctcaaccgcg acgatctcaa 660

ccgcatcaag gcaaaactct tcaccttcga ggatat 696

<210> 32

<211> 1068

<212> DNA

<213> Brucella

<400> 32
atggcggacg atgctgacaa ggaaagcaaa acagaagaag ccaccgagca gaagatccgc 60

gatgccctgg acaagggcaa catgcccttc tcccgcgaag cgcccatcct tgctggcatt 120

gcttcctttc tggtcattgc cgtcttcgtg gcggccccgg cagcaaccag gctcgcggtc 180

ttcctgcgcg aactgatcga ccgcccggaa gactggctct tgaacagtgc tgaagatgcg 240

acccgtcttt tcagcgtgct ggctctcgcc gtcggtgcgg ctttggtgcc ggtcttcatc 300

atcattccgc ttgcgggcat cgcagcctcc gcctttcaga acgcaccccg cttcgtcggc 360

gagcgcatcc gcccgcaggc ctcgcgcatt tcgccgctca agggctggca gcgcattttc 420

gggcgcgccg gccaggtgga attcctgaaa tcgctggcca agcttctggc cgcaagcgtc 480

atcgtctttc tggttttctt caagggaaac agccttttca ccgatgccgt cgccaccgat 540

ccgggtgcgc tgccggaatt cctgcgcaaa aacgtggttc gtcttctggt ggccaatgtg 600

ctcgccatcg cggccattgc cggtttcgat cttgcctggt cgcgcattca ctggcggcag 660

gaattgcgca tgacccggca ggaggtgaag gatgaattga aacagtcgga aggcgatccg 720

ctggtcaagt cacgcctgcg ctcgcttggc cgcgaccggg cacgccgccg catgattaac 780

gctgtaccga ccgccacgct gattgtcgca aacccgaccc acttctcggt cgccttgcgt 840

tacaagccca atgaagatgc cgcccccgtg gtcgttgcca aggggcagga tctgatcgcg 900

ctcaagataa gggaaattgc agcatcccat tcgattcccg tcttcgagga tgtacaattg 960

gcgcgtgcac tctataagca ggtcaatgtc gatcagatga tcgcgcccga gttctacaag 1020
gccgttgccg aactcatccg catcatcaac acacggcgcg caataagt 1068

<210> 33
<211> 531
<212> DNA
<213> Brucella

<400> 33
atgaagagct tgcagttttc aaaagagaga gaaaccatca tcgcggaaaa tatccgggag 60
gtcgtttctg atctccggct ggttgatccc gcagattata ttgccttcat acgctgtgag 120
ctctttgcca atattgccga tcttgtaagc tctgctgccg agctctattt ctatcccggc 180
acactgggcc tcggtcacgg cggtgaattt cattgcgact ggcagtcacc gccttccatc 240
accctcgaca tggaatttcg ccatcagggc gtctatgcct attttcgcat taccctgacg 300
gacaggacgg caggcgtgga attgaaccat atcgccttcg aggatgcgga tggcgatcct 360
tccgccaaca cagtgcggct ggcgaaagct tttgaggatg cccggttgcg cccctgcgca 420
aacgcgcctg aaacgcgtct gagcttttct tttcgcctgc tacggcgtcg cgcggaaggt 480
ttccgcgcga ccatgttgat cagacgagat gctgtacctc gggcatatcg a 531

<210> 34
<211> 414
<212> DNA
<213> Brucella

<400> 34
atgcttgcag cttgcgggaa accgatgaat atcatggctt tgatttcgat tctccaccta 60
tcgagtggcg gggtggcgct tgccgctgat acgggccgca tcattatcag gaacgatctg 120
gccgcatgtg tcgatatgcg agtggaaaag cccccgtttg aatattccaa tgggtcgaat 180
atcgtcgcga ttaacgcgaa ttttaagctg cgaaagccga taggcgcatg tgggtgcatg 240
tcggcactgg caagatatgc cagcagcgtg aatgagcggg aaagccggct gttccttcaa 300
caaggtttgt tcaacctgaa aaagagcgac accaaaacgc tgccccttgc gactgaacct 360
gccttggtca aagacgggaa tatcgagata acggtagggt gtgcgcggcc aaga 414

<210> 35
<211> 492
<212> DNA
<213> Brucella

<400>  35
gtggttccag cggttctgtt aaaacaggaa tcactctacc ttctgcaagt ttcgtttgat      60
aattcatcag cagatataac ggcgaagcaa tatacgaaga ggcactatat ggattttcct     120
gcgtggacct ggtgggcggt ggcgataggt cttttccttc tgatcgaagc gctaatggcc     180
atctggtata ttcgtaggct atctcggcaa cgtgttgaga caactgcggc cctgaaagag     240
gtccaacata ccggtgagcc cggttctgcc ttggttctgg aagccaccga tacaggcaag     300
cggcttggtg catacagttt tttagttgtg cgcctgcgct tgcgcgtgac gcgcgccaat     360
ggcgaggaat ttgaaacggc gattgacgct catatttctc cggtgcgaat tgcggatttt     420
gccgaaggca aacacataaa ggtcagcatt gatcctcatg ccagaacagt ggttttgaac     480
caacctgtac aa                                                         492

<210>  36
<211>  738
<212>  DNA
<213>  Brucella

<400>  36
atgaagcact ccctcggtct cggcgtcatc ctgacgctcg ctctcaccgt ttccgcacag      60
gcgcagcagg cgctgccgct cgacaagctt ctgcccgcgg gcagcggggc tgcaagcggc     120
cagatcgtgc aattgttcgg gcttttgacc gtgctttcca ttgcgccggg cctgcttatc     180
atggtgacaa gcttcacgcg ctttgccatt gccttctcgc ttttgcgttc gggccttggt     240
ctccagaccg cacctgccag catggtaatg atctcgcttg ccctgttcat gaccttctac     300
gtcatggcgc ccgccttcga ccgggcctgg aacaacggcg tgcagcccct gatgcgtaat     360
gaaatcatgc aggaagcagc cttcggtgaa atatcgcagc ctttccggga gttcatgatg     420
gcacaggtgc gcgacaagga tctgcgcctg tttgaagacc ttgccgatcc gtcgtttcgc     480
acgagcgatg acggcatcgt cgatttccgg gtgctggttc ccgcattcat gatttcggaa     540
ctgcggcgcg gttttgaaat cggcttcctg atcgtgctgc catttctggt gatcgacctc     600
gtggtagcga cgctcaccat gtccatgggt atgatgatgc tgccgccgac ggtgatatcg     660
ctgccgttca agattctctt cttcgtgctg attgatggct ggaacattct ggttggcagc     720
ctgatccggt ctttttcg                                                   738

<210>  37
<211>  489
<212>  DNA
<213>  Brucella

<400>  37

atgagtgatg cggcacggga aaaggatggc aaggcgaagg gttccatgat gggcctgctt 60

gcggctgtcg ccgttctcac ggcaattgcc ggcggtggcg gctggtatct gggcaatatg 120

atttccgccg ggcaacaggc ttccgccaaa ccttccggca aggaagacaa gccaaagccg 180

ggaagcttcg agtcccgctc catcggtgcg gttgtgccgt tgcagccgat catcaccaat 240

ctgggcattc cacagaccac ctgggtgcgg ctcgaagcct cgcttgtcgc caaacccggc 300

cacgaaattc cgccggcaat ggctgccaat gtcagcgatg atttcatgcg ttttctgcgc 360

agcgtcaatc tcatgcaatt gcagggcgcg gcggggcttg cctatctgcg caccgatctg 420

gaagaacgcg cgcgcctgcg ttcagaaggc gcggttgacc gtgttttcat ctcggcgctg 480

gtggtggaa 489

<210> 38

<211> 726

<212> DNA

<213> Brucella

<400> 38
atgaaaaaca gcatgaacaa agcgatcctt gcagtagcaa tggtgctcct tctggccggt 60

tgcgccacca agcccgagga aattggccgc gcgcccgacc tgtcgccggt cgcggcccat 120

ctcggcatgc agaacaatcc gcagttcaac ggctatccgg cgcggcccgg caaggcgtcc 180

tattcgctct gggatcagcg ttcgagcaat ttcttcaagg acccgcgcgc ggcgacgccg 240

ggcgatgtgc tgaccgtcat catttccatc aatgatcgcg ccaacctcga taacaagacg 300

gaccgcgagc gcgtgtcgaa gggcatttac ggcggaggcg gttcattcgc caccagcagc 360

attacgggcg cggcggctgg cggtgatatg gatgcttcgg tcaacactca ttccgacagc 420

aagtcgaagg gcaagggcac gattgaacgc agcgaggata tccgcttgca gattgccgcc 480

atcgtcaccg acacgctgcc gaacggcaac ctcatcatcc gtgggtcaca ggaagtgcgt 540

gtgaacaatg aattgcgcgt gctgaacgtt gccggtgtcg tgcgtccgcg cgatatttcc 600

ggcaacaaca cgatttccta cgacaagatc gccgaagcgc gcatctccta tggcgggcgc 660

gggcgactga gcgagattca gcagccgcct tatggccagc agattctcga ccagttttct 720

cctttc 726

<210> 39

<211> 558

<212> DNA

<213> Brucella

<400> 39
atgattatgg cgcataaaat gatccggtat ggttcggcgg tggcgctgtt tctcgcggcg 60

tctgttcccg cttttgccga tccgcccgcg ctcggcaatc tcgatgaaat ccgcaagttc 120
tgtggcaata tcgacgatca ggcggcagat gcccgctatg ccttgcaggc caaacaattg 180
accgaactga aggccgatgt cgaggaacgc atgcgcgcgc ttgaggaaaa gcgccgtgaa 240
tatgaattgt ggctgaagcg ccgcgacgag tttgtcagca aggcacagga tgcactggtc 300
gatatcatct cgaaaatgaa gccggatgcg gccgctgcgc aaatggcgct gatcggtgat 360
gaagcggcag cggcacttat cctcgagctc aatccgcgcg tatccagcat catcctcaat 420
gaaatgccgc cggaaaaggc ggccaagctt gcgcgcgtca ttgtcggttc gcagcgcacg 480
tcaagtgctg cgcagcctgt gaaggatcag tcgggcaggg atcagcgcgc cgcagcgcag 540
accgggaata cggtgcaa 558

<210> 40
<211> 1071
<212> DNA
<213> Brucella

<400> 40
atgacctatg ccgagcttgg ccggggcagc gcggttgcgc gcctgaagga catcgccacc 60
attcagggcg tgcgcgaaaa ccagcttgtg ggctatggtc ttgtaatcgg tctcaagggc 120
accggcgaca gcctgcgcaa ttcgccgttt accgaacaat ccatgcgcgc catgctggat 180
aatctgggca tcagtgcacc gcgcaattcc acccgctcga agaatacggc ggcagttatt 240
gtgacggcta accttcctgc ctttgccggt gccggttcgc gcatcgatgt aacggtgtcg 300
tcacttggcg atgcgacctc gcttcaaggt ggtacgcttg tcatgaccca gctcatgggg 360
gcggataatc agatttatgc cgtggcgcag ggcaatatga tcgtctccgg cttttcggcg 420
gaaggtgaag ccgcaagcgt cacgcaaggc gtgccaactt ccgggcgcat ccccaatggc 480
gcgctggtcg agcgcgaggt ggcgggcagt ttcggcaagg accgggagat gatcgtggaa 540
ctgcgtgacc ccgatttcac gaccgccgtg cgcgctgccg atacgatcaa tgtcttcgcc 600
aagcgccgtt atggtcgcgg ggtggccatt gcgcgcgatg ccaagacgat ccgcctgtca 660
cgcccgaaga acgtgcctgc cgcccgtttt cttgcagagc ttgagggcct gccgatcacc 720
accgacgaag tggcgcgggt ggtagtggat gagcggaccg gaacggtcgt tattggcgaa 780
aaggtgcgca tttccaaggt tgcgatcagc catggcagcc tgacggtccg tgtgacggaa 840
acgccgatgg tcgtccagcc agagccgttc agtgacggcg agacggcggt tgagcccaac 900
accgatatcg ccgtcaacca gcagaacgcc aagatcggca tcctgagcgg cgccaatctc 960
gaaaatctcg tgaaggggct caatcagatc ggcgtgaagc ccaccggcat catcgcgata 1020
ttgcaggcaa tcaagacatc cggcgcgctt catgccgaac tggtggtgca a 1071

<210> 41
<211> 411

<212>  DNA
<213>  Brucella

<400>  41
atggctggcg ccggtgcggc agaccgcatt gcttttgtcg tgccttcggc gaccgtctat      60
cccggaccaa tcgtgagcga taccgcgctt ctggaaaagc agttcttcat caatgcgcct     120
gcggcgaccc aatatgtgct gtcgctggat caggcggtgg gcaaggtggc gcgcaagacg     180
ctgcttcccg gcaagccgat cctcgtttcg gcactgggcg agccgtctct ggtcacgcgc     240
ggcattcctg caccactggt ctttaccgcc ggcacgctga ctataactgc gatgggtaca     300
ccgctcgaat ccggtgtggc tggcgatttc atcaaggttc gtaacgtcga tagcggcatc     360
atcgtgtccg gcacagtgct cgcaaatggt cgtattcagg tggggatgca g              411

<210>  42
<211>  786
<212>  DNA
<213>  Brucella

<400>  42
atgaaagccc tgacgattgc cgccacaggc atgaacgccc agcagcttaa tctggaagtg      60
attgccaaca atattgccaa tatcaacaca acgggcttca agcgcgcacg ggccgaattt     120
accgatcttc tctaccagtc ggagcgcacg gcgggcgtgc ccaatcaggc gaaccaggcc     180
atcgtgcccg agggcgcgct ggtgggcctt ggcgtgcaga cggcggcggt gcgcaatctc     240
cacattcagg gcagtttcaa ccagaccggc aatccttatg acgtggcgct gaccgggcgt     300
ggctggttcc agatccagag cccgaccggc gagacgcttt atacccgcgc tggcgcattc     360
aacaagaatg ccgatggcca gcttgtgacg cttgacggca atccggtcga accggccatc     420
accattccgc cggacgctat tgaagtgacg atcaccgata cggggcaggt tttcgccaag     480
ttgcaggacc agatcaatca ggttgatctc ggccagttaa cccttgcaaa tttcaccaat     540
gaagccggtc tcgaaccgct tggcggcaat ctctaccgcg agacggaagc atccggcggc     600
ccgctggtcg gcgttccggg tgatccgggc tacggctcga tcaagcaggg ctatctggaa     660
gcgtcgaacg tcgatccggt caaggaaatt accgatctca tcacggcgca gcgcgcttat     720
gaaatgaact ccaaggttat tcaggccgcc gacgaaatgg cggcgaccgt atcgaagaac     780
ttgcgt                                                                786

<210>  43
<211>  333
<212>  DNA
<213>  Brucella

<400> 43
atgtacgact cgatcatgag tgtttcggcg cgcaatgccc tgtcgcgcct gtcggaaacc 60

gttgccgaaa agggcgttgg gtccgcttcc gcaccgcagg ccgttccggc cgcccccggt 120

gcttccttcg gtgaagttct gtcgcagatg acgggttccg tcagccagaa attgcaggcg 180

gcagaggcaa cgtcgataca gggcatcaag ggcgatgcgc cggtgcgcga tgtggtcagt 240

tccgtcatgg aagccgaaca gtcgctccag acggccatcg cgatccgcga caagatcgtg 300

caggcctatc tcgaaatcag ccgtatgccc atc 333

<210> 44

<211> 420

<212> DNA

<213> Brucella

<400> 44
atgtcttccg atgccttgca aagcacattg aagatcgcgg catcgggcct ttcggcccag 60

tcgacacgcc tgcgcatcgt ttccgaaaat attgccaatg cgcagtccac cgccaggacg 120

gcggataccg atccctatcg ccgcaagacg gtttcgttcc gcaccgagct tgaacaaggc 180

acgggtgcag ccgaggttcg ggttgcggaa gtgggcaagg atacctcgcc ctttatcgaa 240

caatatgatc cgagccatcc cgcagcggat gcgcgcggct atgtcaaata tccaaatgtc 300

aacatggtcg tcgagatggc ggacatgcgc gaggcaaacc gttcctacga agccaatttg 360

caggtggtca ggcaggcgcg tgagctgatt tcgatgacga tcgacctctt gaggagcaca 420

<210> 45

<211> 378

<212> DNA

<213> Brucella

<400> 45
atgagctcca ttcatctttt cgatctggct gcaaggcagg cgcagtggtt gtcggtgaga 60

caggccaccg tcgcaggcaa tgtcgccaat gcgaatacgc cggatttccg ggcgcgggat 120

gtgcagccct tcgcggatgt gctggataag acgcagctta ccatgactgc caccagcccc 180

ttgcatctgg aagccgaggc ggacggtatc gcggccacgc gcctgcgccc cgatgacgtt 240

accgagcaga cccattccgg caatacggtc gatgttgaac aggaaatgat gaaggctggc 300

gaaatcgccc gcgaatattc gctcaacacc agcatcgtca aggcgttcca tcgcatgatg 360

ctttcggtcg cgaagggg 378

<210> 46

<211>  777

<212>  DNA

<213>  Brucella


<400>  46
atgagtcttg atctcgttca ttttccgaat tacaaaaaaa ccttttttgg ttcttccttt      60

caaagcgata ctttggcgct tctaacccgc atccgggatg aaataggatg ccgctacgta      120

acgcatacct atcgtggtcg cgtaggcgac tgcacgaagg tgaattcagc cgatctgact      180

gttcttatga cgctgcctgc tacctgggtc gcgcgctatt cctcgaaaaa ctactttgcc      240

attgacccgg tttttcagga agacgctccg tattaccgca atgatacgag cgccattgcc      300

cgcgacctga aagaagatgc ggatatttgc ccggccgttg ccgaactgtt gcatgatgct      360

gaaaagcacg gcctgggaaa tcttttcatc gccgtatccg cgcgcaatcc caagggcgtt      420

gcgggttgta cggttttcac tttcgaggtg gaggacgaag accgtacgca attccttgcc      480

aggatgcgcc cgcgccttct aagccttgcc ggcatcattc atggaactgt ctgcggctgc      540

aaggatgcaa attcagttgc aagccttctc accccgcgcg aggtcgattg cctgcgctgg      600

gctgccaatg gcaagactga tggcgaaatc gccgaaatcc tcagcatcgc ccgctggacg      660

gtggtgactt atctacagaa tgccaaaatc aagctgaact gttccaatcg aacgtcggca      720

gtggcgacgg cgctttccct cggcattatc gatatgcccg aggtacagca tctcgtc      777

<210>  47

<211>  741

<212>  DNA

<213>  Brucella


<400>  47
atgaaacctg cacgcaacac ttcccgcatt ggcgacattt ccgacgacaa gaaaggcgag      60

cttcgttcag ccccggacaa ggccgaatta tccgcctcct gcctgcctga aaccgaatgt      120

gggcgcacaa ggcttgctgc ggggcaggac ccggccaagc gcagccagat cctcgaaggc      180

gcgcaaagcg tgttcctgcg catggggttc gatgccgcca gcatgaacga catcacccgc      240

gaggcgggcg tatcgaaggg aacgatctac gtctatttca acagtaagga agacctgttt      300

gtcgcgctat gcgaacatta tcgccagacg ctgttcagca gctttatcgg ccagttggaa      360

aagggctttt ccaaccgcca ggagctgatc gatttcggcg tggcgctgac gacgctgatt      420

acctcctcca tcgccattcg cgcgcagcgc atcgtcgtcg gtgtgagcga gcgcaagccg      480

gaacttgcag cacgctttta cgagcgcggc ccgaaacgca gccatgccat catggcgcaa      540

tcccttcagg cgatgatcga cgcgggtgtg ctcgagcagc acgatgtaac aagaacagcc      600

tatcaacttt ctgatctttt tttcgccggg ctctatcgtc cccggctctt cggtgcgctt      660

gcggaggcac cttccgagca ggcgattcgg gacaatgtgg agtccgcggt agactttttc 720
tttagagcct atggcaaaaa g 741

<210> 48
<211> 792
<212> DNA
<213> Brucella

<400> 48
ttgtttgaag ggcagtcggt gcttcggtcg gaaaaagagt tgctggagca gcgggtgcgc 60
gagcgcacgg ccgaactgga gcaggagcgc agcattgccg agcgtgagcg caagcgggtg 120
gaggttctcc ttcaggatac caatcaccgc attggcaatt cgctggcgac ggtttcatcg 180
cttctgggcc tgcaaatgcg ccagacgcag gatgaaaatg cgcgtgcggc actggttgcg 240
gcgcgcgacc gggtgcagac ggtttccacc gcgcatcgcc ggcttcggct tggcgaggat 300
atggaaaccg cacgggtgga tgaattcctg caatcggtga tcggggatat ccgcagcgca 360
attgggcatg accgcgatat tcggttcgag actgattttt cgccgcttga tctgaaagcg 420
cgcgatgtca cgacaatcgg catcgttctg ggagaactca ttaccaatgc catcaaacat 480
gcttttcccg gacgcaaaca agggcgtatc agcgtcagtc tcaagcccga tatggagaat 540
gttccggtac tgcttgttga ggatgacggt gttggctggc aacggaaagc gggcgatgac 600
aagcgggtaa acgggcgggt aaacgggctt ggcatgattg tcgttgaaca gctttgcctg 660
caattcggtg aaaagccggt ttatgggcag gcggaaaata gctgcggaac gcgggtgacc 720
gttcgcctct catcgctggt ggaagaacag caggacgaga cggcgagtga agccgaagat 780
gccgctgcaa aa 792

<210> 49
<211> 570
<212> DNA
<213> Brucella

<400> 49
gtgggcagaa caacaggtcc gtcggttgag gggacgccgc cgttcaagcg tgaacttctg 60
gcgtcgctgc cgagcttgcg cgcatttgcg gtctcgctga tcggtcagca tgaccgggcg 120
gacgatctcg ttcaggatac gatcatgaag gcatgggcca agcaggagtc ctttgaagtg 180
gggaccaata tgaaggcttg gctcttcacc attctgcgca atgaattcta cacccagatg 240
cgcaagcgcg gccgcgaggt gcaggatacg gacggcatgt tcagcgaaca gcttgccgtg 300
catccctcgc aatatggcac gcttgatctt caggattttc gcgctgccct cgaccagctt 360
ccggacgacc agcgtgaggc aatcatcctc atcggggctt cgggctttgc ctatgaggaa 420

gcggccgaga tttgcggctg tgccgtgggc acgatcaaaa gccgtgtcag ccgcgcccgc 480

gcccgccttc aggaaattct ccagatcgaa ggcgaggcgg attatggccc cgacgccgat 540

tcctcccgtg cgacccccag aagcttcgcc 570

<210> 50

<211> 792

<212> DNA

<213> Brucella

<400> 50
atgacgttat cgacgcgtat agcgccgcat cttccctatt tgcgccgttt ttcccgcgcg 60

cttaccggat cccagtcctc tggcgatgcc tatgtagccg cagcgctgga agccctgatt 120

gccgatgtcg gcatcttccc cgaagcttct tccgatcgga tcggtctcta caggcttttt 180

tgcaatctct ataaaaatgc ctccatccgc atgcccgaaa cctcttcgga attcgcctgg 240

gaaacccatg cagcgcgcaa tctggcccat attgcccctt tgccgcgcca ggctttcctg 300

ctggtcgccg tggaaggctt caacgatcat gaggcagcag aaatccttga agtggaccaa 360

gcggaattcg gacggcttct ctccaccgcc tccggcgaaa tttcacgcca ggtggcaaca 420

cgtctgatga tcattgagga tgaacccctc attgccatgg atatcgaaca gatggtcgaa 480

agcctcggcc atgaagtggt tggtattgcg cgcaccaagg atgaggcgct ggcgctttat 540

gaaaaagaaa agccgcgcat ggttctcgcc gatatccagc ttgccgacgg tagctccggc 600

atcgatgcgg tcaatgagat acttcacgac aacaccatcc cggtgatttt catcaccgca 660

tttccggagc gcctgctgac gggcgagcgg cccgagccga catttctcgt caccaagccg 720

ttcaaccccg acatggtgaa ggccctcatc agccaggcac ttttctttga ggaagcatcg 780

caagttgctg cc 792

<210> 51

<211> 15

<212> DNA

<213> Brucella

<220>

<221> misc_feature

<222> (5)..(11)

<223> any nucleotide

<400> 51
ttaannnnnn nttaa 15

<210> 52

<211> 18

<212> DNA

<213> Artificial sequence

<220>

<223> upflgI primer

<400> 52
gcgcgcctga aggacatc 18

<210> 53

<211> 18

<212> DNA

<213> Artificial sequence

<220>

<223> lowflgI primer

<400> 53
acggcggtcg tgaaatcg 18

<210> 54

<211> 27

<212> DNA

<213> Artificial sequence

<220>

<223> kanamont primer

<400> 54
gggcatgcgg aaagccacgt tgtgtct 27

<210> 55

<211> 30

<212> DNA

<213> Artificial sequence

<220>

<223> kanaval primer

<400> 55

ggggtgacct tagaaaaact catcgagcat 30

<210> 56

<211> 533

<212> DNA

<213> Artificial sequence

<220>

<223> flgI amplicon

<400> 56
gcgcgcctga aggacatcgc caccattcag ggcgtgcgcg aaaaccagct tgtgggctat 60

ggtcttgtaa tcggtctcaa gggcaccggc gacagcctgc gcaattcgcc gtttaccgaa 120

caatccatgc gcgccatgct ggataatctg ggcatcagtg caccgcgcaa ttccacccgc 180

tcgaagaata cggcggcagt tattgtgacg gctaaccttc ctgcctttgc cggtgccggt 240

tcgcgcatcg atgtaacggt gtcgtcactt ggcgatgcga cctcgcttca aggtggtacg 300

cttgtcatga cccagctcat gggggcggat aatcagattt atgccgtggc gcagggcaat 360

atgatcgtct ccggcttttc ggcggaaggt gaagccgcaa gcgtcacgca aggcgtgcca 420

acttccgggc gcatccccaa tggcgcgctg gtcgagcgcg aggtggcggg cagtttcggc 480

aaggaccggg agatgatcgt ggaactgcgt gaccccgatt tcacgaccgc cgt 533

<210> 57

<211> 1071

<212> DNA

<213> Brucella

<400> 57
atgacctatg ccgagcttgg ccggggcagc gcggttgcgc gcctgaagga catcgccacc 60

attcagggcg tgcgcgaaaa ccagcttgtg ggctatggtc ttgtaatcgg tctcaagggc 120

accggcgaca gcctgcgcaa ttcgccgttt accgaacaat ccatgcgcgc catgctggat 180

aatctgggca tcagtgcacc gcgcaattcc acccgctcga agaatacggc ggcagttatt 240

gtgacggcta accttcctgc ctttgccggt gccggttcgc gcatcgatgt aacggtgtcg 300

tcacttggcg atgcgacctc gcttcaaggt ggtacgcttg tcatgaccca gctcatgggg 360

gcggataatc agatttatgc cgtggcgcag ggcaatatga tcgtctccgg cttttcggcg 420

gaaggtgaag ccgcaagcgt cacgcaaggc gtgccaactt ccgggcgcat ccccaatggc 480

gcgctggtcg agcgcgaggt ggcgggcagt ttcggcaagg accgggagat gatcgtggaa 540

ctgcgtgacc ccgatttcac gaccgccgtg cgcgctgccg atacgatcaa tgtcttcgcc 600

aagcgccgtt atggtcgcgg ggtggccatt gcgcgcgatg ccaagacgat ccgcctgtca 660

cgcccgaaga acgtgcctgc cgcccgtttt cttgcagagc ttgagggcct gccgatcacc 720
accgacgaag tggcgcgggt ggtagtggat gagcggaccg gaacggtcgt tattggcgaa 780
aaggtgcgca tttccaaggt tgcgatcagc catggcagcc tgacggtccg tgtgacggaa 840
acgccgatgg tcgtccagcc agagccgttc agtgacggcg agacggcggt tgagcccaac 900
accgatatcg ccgtcaacca gcagaacgcc aagatcggca tcctgagcgg cgccaatctc 960
gaaaatctcg tgaaggggct caatcagatc ggcgtgaagc ccaccggcat catcgcgata 1020
ttgcaggcaa tcaagacatc cggcgcgctt catgccgaac tggtggtgca a 1071

<210> 58
<211> 569
<212> DNA
<213> Brucella

<400> 58
atgacctatg ccgagcttgg ccggggcagc gcggttgcgc gcctgaagga catcgccacc 60
attcagggcg tgcgcgaaaa ccagcttgtg ggctatggtc ttgtaatcgg tctcaagggc 120
accggcgaca gcctgcgcaa ttcgccgttt accgaacaat ccatgcgcgc catgctggat 180
aatctgggca tcagtgcacc gcgcaattcc acccgctcga agaatacggc ggcagttatt 240
gtgacggcta accttcctgc ctttgccggt gccggttcgc gcatcgatgt aacggtgtcg 300
tcacttggcg atgcgacctc gcttcaaggt ggtacgcttg tcatgaccca gctcatgggg 360
gcggataatc agatttatgc cgtggcgcag ggcaatatga tcgtctccgg cttttcggcg 420
gaaggtgaag ccgcaagcgt cacgcaaggc gtgccaactt ccgggcgcat ccccaatggc 480
gcgctggtcg agcgcgaggt ggcgggcagt ttcggcaagg accgggagat gatcgtggaa 540
ctgcgtgacc ccgatttcac gaccgccgt 569

<210> 59
<211> 1035
<212> DNA
<213> Brucella

<400> 59
gcgcgcctga aggacatcgc caccattcag ggcgtgcgcg aaaaccagct tgtgggctat 60
ggtcttgtaa tcggtctcaa gggcaccggc gacagcctgc gcaattcgcc gtttaccgaa 120
caatccatgc gcgccatgct ggataatctg ggcatcagtg caccgcgcaa ttccacccgc 180
tcgaagaata cggcggcagt tattgtgacg gctaaccttc ctgcctttgc cggtgccggt 240
tcgcgcatcg atgtaacggt gtcgtcactt ggcgatgcga cctcgcttca aggtggtacg 300
cttgtcatga cccagctcat gggggcggat aatcagattt atgccgtggc gcagggcaat 360

atgatcgtct ccggcttttc ggcggaaggt gaagccgcaa gcgtcacgca aggcgtgcca 420
acttccgggc gcatccccaa tggcgcgctg gtcgagcgcg aggtggcggg cagtttcggc 480
aaggaccggg agatgatcgt ggaactgcgt gaccccgatt tcacgaccgc cgtgcgcgct 540
gccgatacga tcaatgtctt cgccaagcgc cgttatggtc gcggggtggc cattgcgcgc 600
gatgccaaga cgatccgcct gtcacgcccg aagaacgtgc ctgccgcccg ttttcttgca 660
gagcttgagg gcctgccgat caccaccgac gaagtggcgc gggtggtagt ggatgagcgg 720
accggaacgg tcgttattgg cgaaaaggtg cgcatttcca aggttgcgat cagccatggc 780
agcctgacgg tccgtgtgac ggaaacgccg atggtcgtcc agccagagcc gttcagtgac 840
ggcgagacgg cggttgagcc caacaccgat atcgccgtca accagcagaa cgccaagatc 900
ggcatcctga gcggcgccaa tctcgaaaat ctcgtgaagg ggctcaatca gatcggcgtg 960
aagcccaccg gcatcatcgc gatattgcag gcaatcaaga catccggcgc gcttcatgcc 1020
gaactggtgg tgcaa 1035

## Description

### Objet de l'invention

La présente invention est relative à des nouvelles séquences nucléotidiques encodant des protéines flagellaires de Brucella importantes au niveau du cycle infectieux de ce micro-organisme chez les mammifères.

La présente invention concerne également les protéines encodées par ces séquences nucléotidiques et les applications diagnostiques de ces séquences génétiques, en particulier une composition pharmaceutique ou une trousse de diagnostic comprenant ces séquences génétiques ou des molécules inhibitrices, en particulier des anticorps ou des séquences anti-sens dirigés contre ces séquences.

### Arrière-plan technologique à la base de l'invention Brucella et brucellose

Les bactéries du genre Brucella sont des coccobacilles gram-négatif qui appartiennent à la famille des α-Protéobactéries. Ce sont des pathogènes intracellulaires cultivables en milieu de laboratoire, mais dont on ne connaît pas de forme libre naturelle. Différentes espèces ont été définies en fonction de leur propension à être associées à un hôte particulier (pour revue [Moreno, 2002 #160]). On distingue *B. melitensis chez les caprins, B. abortus* chez les bovins, *B. canis* chez le chien, *B. suis* chez le porc, *B. ovis* chez les ovins et *B. neotomae* chez certains rongeurs. Deux nouvelles espèces associées aux mammifères marins sont en cours de description, *B. cetaceae* et *B. pinnipediae* [Cloeckaert, 2001 #161].

Chez les animaux, la brucellose est une maladie du système reproducteur induisant des avortements chez les femelles gestantes et des orchites chez les mâles. La transmission d'un animal à l'autre semble majoritairement due aux avortements qui libèrent un nombre élevé de bactéries dans l'environnement. La transmission à l'homme s'effectue soit par contact direct avec des avortons infectés, soit par consommation de produits laitiers non pasteurisés. Chez l'homme, seules les espèces *melitensis*, *suis, abortus* et *canis* sont connues pour induire une infection systémique pouvant prendre des formes variées (méningites, endocardites, arthrites ...) et souvent associée à une fièvre ondulante, appelée fièvre de Malte.

Les processus par lesquels *Brucella* infecte son hôte sont encore mal connus. Elle pourrait pénétrer passivement via des ruptures de l'intégrité de l'épithélium, mais aussi en traversant les muqueuses respiratoires et/ou digestives. Une fois dans les tissus sous-jacents, la bactérie fera face au système immunitaire de son hôte. Pour s'en prémunir, *Brucella* a développé une stratégie originale qui consiste à se « cacher » au sein même de celui-ci. La bactérie utilise en effet les cellules phagocytaires comme première niche de réplication. Les cellules immunitaires deviendront donc des chevaux de Troie permettant la dissémination de la bactérie au sein de tout l'organisme. Chez les animaux, *Brucella* est le plus souvent associée aux tissus lymphoïdes, à la glande mammaire et aux organes reproducteurs. Chez les femelles gestantes, Brucella envahit les cellules du trophoblaste chorioallantoique, qui constituent sa deuxième niche de réplication. La réplication massive de la bactérie au sein du réticulum endoplasmique rugueux (RER) va entraîner la nécrose cellulaire et l'ulcération de la membrane chorioallantoique [Anderson, 1986 #166]. L'infection se répandra alors dans les tissus foetaux, provoquant *in fine* l'avortement.

### Les déterminants moléculaires de la virulence de Brucella

### [Bosschiroli, 2001#167;Ko,2003#164]

La pathogénie induite par *Brucella* repose donc principalement sur sa capacité à se répliquer dans les cellules eucaryotes (phagocytaires et non-phagocytaires). Les mécanismes moléculaires impliqués dans ces processus ont fait l'objet de nombreuses études. Celles-ci, en utilisant des approches variées (délétion, mutagénèse transpositionnelle, DFI, STM...) dans divers modèles d'infections (macrophages de différentes espèces, cellules HeLa, souris BALB/C et C57 Black, chèvres ...) ont permis l'identification de plus de 200 gènes de virulence. L'analyse de ceux-ci permet d'inférer les étapes cruciales du cycle infectieux de *Brucella*. Celles-ci, pour plus de clarté, sont divisées en 6 classes [Mahan, 2000#61] : la satisfaction des besoins métaboliques, la réponse au stress, la régulation, l'invasion, la survie intracellulaire et les interactions de surfaces.

### La satisfaction des besoins métaboliques

### A- La synthèse des précurseurs du métabolisme

Le milieu intracellulaire étant pauvre en nutriments accessibles aux bactéries, elles doivent être capables de synthétiser de novo certains précurseurs de leur métabolisme [Finlay, 1997#41]. Suite à l'analyse des différents cribles publiés, on peut en conclure que dans sa niche de réplication, *Brucella* doit au moins synthétiser *de novo,* les purines et les pyrimidines, différents acides aminés (lysine, valine, leucine, isoleucine, serine, thréonine, histidine, cystéine) ainsi que la thiamine et la cobalamine.

### B- L'acquisition de nutriments

Dans sa niche de réplication, *Brucella* doit être capable d'acquérir et d'utiliser différentes sources de carbone et d'azote pour alimenter son métabolisme. De même certains ions métalliques (fer, magnésium, nickel...) sont des cofacteurs indispensables au bon fonctionnement des processus enzymatiques. Dès lors, leur capture revêt une importance primordiale dans le processus infectieux.

Sur base des mutants avirulents identifiés, il semblerait que la croissance intracellulaire de *Brucella* repose sur l'utilisation d'au moins 7 sucres, à savoir : le maltose, le ribose, l'arabinose, le galactose, le glucose, le glycérol et l'érythritol.

Cinq gènes impliqués dans la voie des pentoses phosphates ont également été liés à la virulence (*cbbE*, *pgi*, *rbsK*, *araG*, *rbsA* ). La voie des pentoses phosphates alimente la synthèse nucléique en ribose, elle est aussi importante dans le maintien du potentiel redox de la cellule en produisant du NADPH, ces deux fonctions étant primordiales pour la survie intracellulaire [Lundberg, 1999 #177]. De plus chez *Brucella,* la phosphofructokinase est absente, dès lors l'utilisation de glucose via la glycolyse est impossible et doit passer par la voie des pentoses phosphate [Essenberg, 2002 #233].

La synthèse d'acides aminés et nucléiques nécessite l'apport d'azote. La sélection de souches atténuées mutées dans des gènes impliqués dans l'utilisation directe du NH₄⁺ suggère que celui-ci pourrait être prélevé dans le phagosome [Kohler, 2002 #98]. Cela dit, l'observation que deux systèmes de transport d'acides aminés sont induits en macrophage peut suggérer qu'une partie de l'azote serait également obtenue sous cette forme [Kohler, 1999 #103].

Chez *Brucella*, il a été montré que des gènes impliqués dans le transport du magnésium, du nickel, du zinc ainsi que certains impliqués dans la capture du fer sont nécessaires à la virulence. A ce jour, seuls les mécanismes impliqués dans la capture du fer chez *Brucella* ont été quelque peu documentés.

En milieu pauvre en fer, *Brucella* synthétise un sidérophore, l'acide 2,3-dihydroxybenzoïque (2,3-DHBA) qu'elle utilise ensuite comme source de fer [Lopez-Goni, 1992 #181;Lopez-Goni, 1995 #182]. Cependant, un mutant Δ-*dhbC* (désigné *entC* précédemment) incapable de synthétiser le DHBA n'est atténué ni en macrophages ni dans aucun des modèles murins testés [Bellaire, 1999 #184][Parent, 2002 #183]. Il est par contre incapable de persister chez des chèvres gestantes [Bellaire, 2003 #186]. On suppose que l'incapacité du mutant *Δ-dhbC* à infecter des chèvres gestantes est liée à son inaptitude à utiliser l'érythritol.

### La réponse aux stress

Plusieurs gènes liés aux conditions stressantes rencontrées par la bactérie et importants pour la virulence ont été identifiés. Si ceux-ci sont importants pour la pérennité de la bactérie, certains facteurs de stress servent également de signaux, lui permettant de réguler ses gènes de virulence adéquatement. On sait par exemple que l'acidification des phagosomes contenant *Brucella* est un signal essentiel permettant sa réplication intracellulaire [Porte, 1999 #192].

### La régulation

A ce jour, 5 systèmes à deux composants et 9 régulateurs transcriptionnels ont été liés à la virulence. Dans les systèmes à deux composants, seul le système *BvrR*/*BvrS* a été caractérisé [Lopez-Goni, 2002 #200]. Des mutants dans un des deux composants montrent un phénotype identique et complexe. Ils pénètrent et se répliquent moins bien dans les cellules, ils sont rapidement éliminés de la souris et sont sensibles aux peptides cationiques [Sola-Landa, 1998 #153]. Les phénotypes liés à ces deux composants seront détaillés plus loin dans le texte.

Un seul régulateur transcriptionnel a été étudié à ce jour, *vjbR*. Ce régulateur appartient à la famille LuxR et possède un domaine de fixation aux homosérines lactones (HSL). Les HSL sont des composés diffusibles impliqués dans le phénomène du quorum sensing. Chez *Brucella,* l'expression de l'opéron *virB* (cet opéron encode un système de sécrétion de type IV impliqué dans la survie intracellulaire) est réprimée en présence d'HSL [Taminiau, 2002 #203]. Le régulateur *vjbR* est un régulateur de l'opéron *virB* et est également régulé négativement par les HSL [Delrue, 2002 #202].

Si la capacité de *Brucella* à détourner le trafic intracellulaire d'une cellule à son propre avantage est remarquable, la capacité de celle-ci à supporter un tel intrus parait extraordinaire. En effet, loin de rentrer en apoptose ou en nécrose, les cellules (phagocytaires) infectées continuent à se multiplier. Il faut qu'il en soit ainsi pour que la bactérie puisse s'en servir comme véhicule au sein de l'organisme. On sait maintenant que *Brucella* empêche l'apoptose des cellules *phagocytaires* qu'elle infecte [Gross, 2000 #208]. Bien que le phénomène ne soit encore que peu compris, la chaîne O du LPS joue un rôle important dans ce phénomène. En effet, des mutants rugueux sont incapables d'inhiber la mort cellulaire programmée des cellules qu'ils infectent [Fernandez-Prada, 2003 #213].

### Les interactions de surface

La membrane externe de *Brucella* constitue sa seule interface avec son environnement. C'est au travers de celle-ci qu'elle doit percevoir le milieu qui l'entoure mais aussi interagir avec celui-ci. On a déjà évoqué l'importance de la chaîne-O du LPS dans l'invasion et dans le contrôle du cycle cellulaire des cellules phagocytaires [Fernandez-Prada, 2003 #213] [Porte, 2003 #212]. Le LPS est formé de la chaîne O, du core et du lipide A, son ancre membranaire. Chez *Brucella,* l'acylation du lipide A semble différente dans les mutants *BvrR-BvrS*, et pourrait jouer un rôle dans la résistance aux peptides cationiques mais aussi dans modulation de la réponse de l'hôte [Lopez-Goni, 2002 #200]. En plus du LPS, les protéines de membrane externe (omp) jouent également un rôle dans la virulence. En effet, l'omp10, l'omp19 et l'omp 25 (omp 3a) ont été associées à la virulence [Tibor, 2002 #225] [Edmons, 2001 #226]. On sait par exemple que l'omp 25 (omp 3a) est liée à l'inhibition de la production de *TNF*-α chez des macrophages infectés par B. suis [Jubier-Maurin, 2001 #237]. Chez les mutants *BvrR-BvrS*, l'omp 25 ainsi qu'une omp non encore caractérisée, omp 3b, ne sont plus exprimées [Guzman-Verri, 2002 #201]. Le système *BvrR-BvrS* est donc impliqué dans des remaniements majeurs de la composition de la membrane externe. Loin d'être une cloison inerte, celle-ci doit être considérée comme un facteur de virulence majeur.

*Brucella* est capable d'inhiber la fusion phago-lysosomale et de se répliquer dans une vacuole possédant les caractéristiques du réticulum endoplasmique. Pour parvenir à sa niche de réplication, *Brucella* met en oeuvre différents acteurs moléculaires dont les principaux sont : a) la chaîne-O du LPS qui lui permet de pénétrer dans les phagocytes via les radeaux lipidiques et d'inhiber l'apoptose ; b) le système à deux composants *BvrR-BvrS* impliqué dans l'invasion cellulaire (activation des GTPases), il semble aussi jouer un rôle dans le passage de l'endosome précoce à l'autophagosome et dans la régulation de la composition de la membrane externe c) le système de sécrétion de type IV qui est impliqué dans le passage de l'autophagosome vers la niche de réplication.

De plus, *Brucella* est décrite comme non mobile mais, en 1998, S.Halling (Halling, S. 1998. Microb Comp Genomics. 3(1):21-9) a découvert 3 CDS homologues à des gènes flagellaires dans le génome de *B*.*abortus*.

### Buts de l'invention

La présente invention vise à caractériser, isoler et purifier des nouvelles séquences génétiques spécifiques de Brucella et trouvant des applications dans le domaine thérapeutique ou diagnostique chez les mammifères (en particulier les ruminants), y compris chez l'homme.

Un but particulier de la présente invention est de mettre en évidence parmi ces séquences, des séquences génétiques importantes au niveau du cycle infectieux chez des mammifères, en particulier des séquences génétiques permettant d'améliorer le traitement thérapeutique, en particulier vaccinal et pour le diagnostic de cette pathologie chez les mammifères (ruminants), y compris chez l'homme.

Un dernier but de la présente invention est d'identifier des molécules (inhibitrices) dirigées spécifiquement contre ces séquences génétiques ou des portions de celles-ci, de manière à améliorer le traitement thérapeutique et diagnostique chez les mammifères (ruminants), y compris chez l'homme.

### Eléments caractéristiques de la présente invention

La présente invention concerne en particulier de nouvelles séquences génétiques, en particulier des séquences nucléotidiques encodant des protéines flagellaires de *Brucella*, ainsi que les dites protéines encodées.

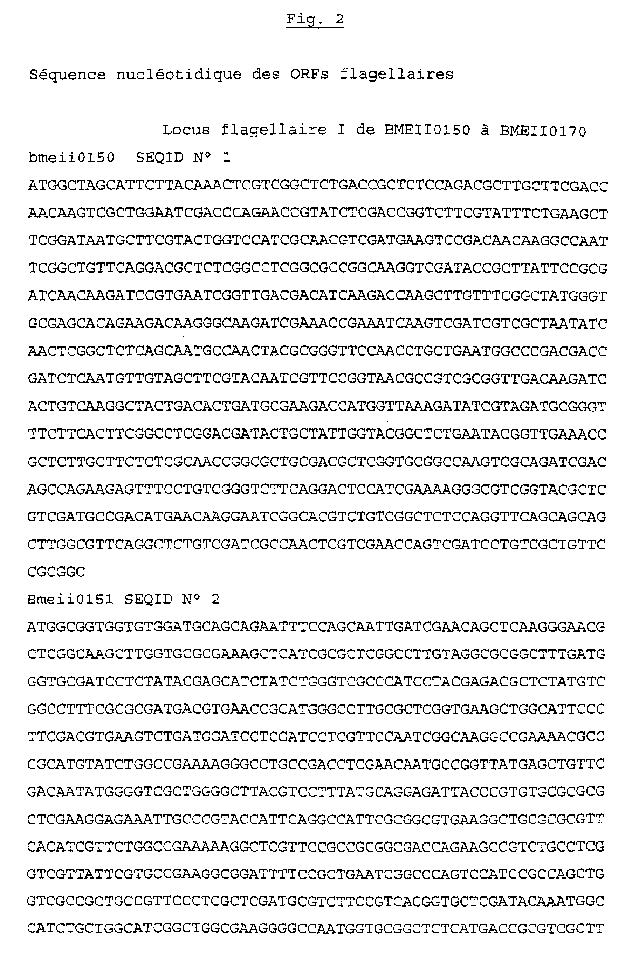

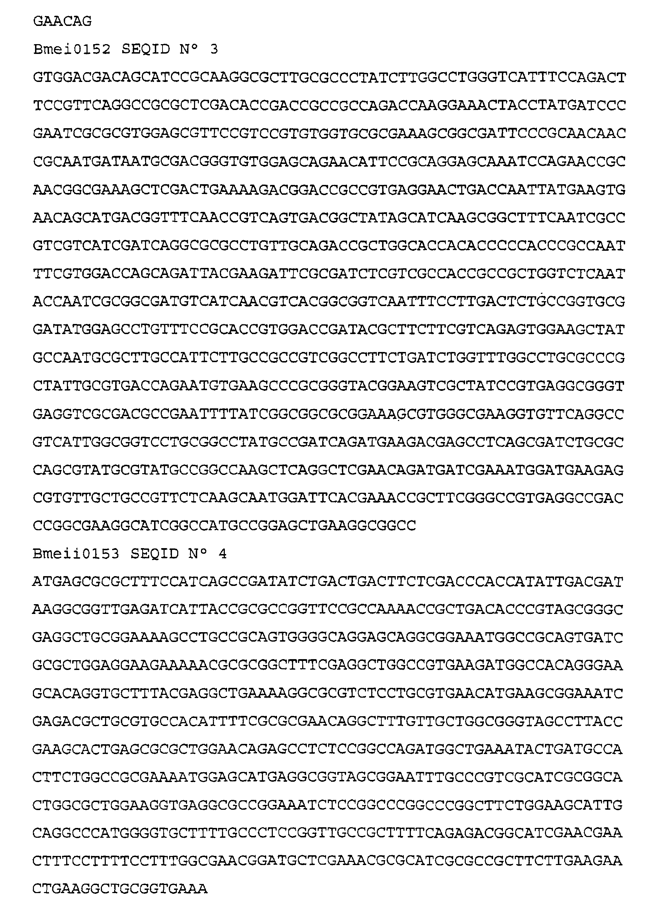

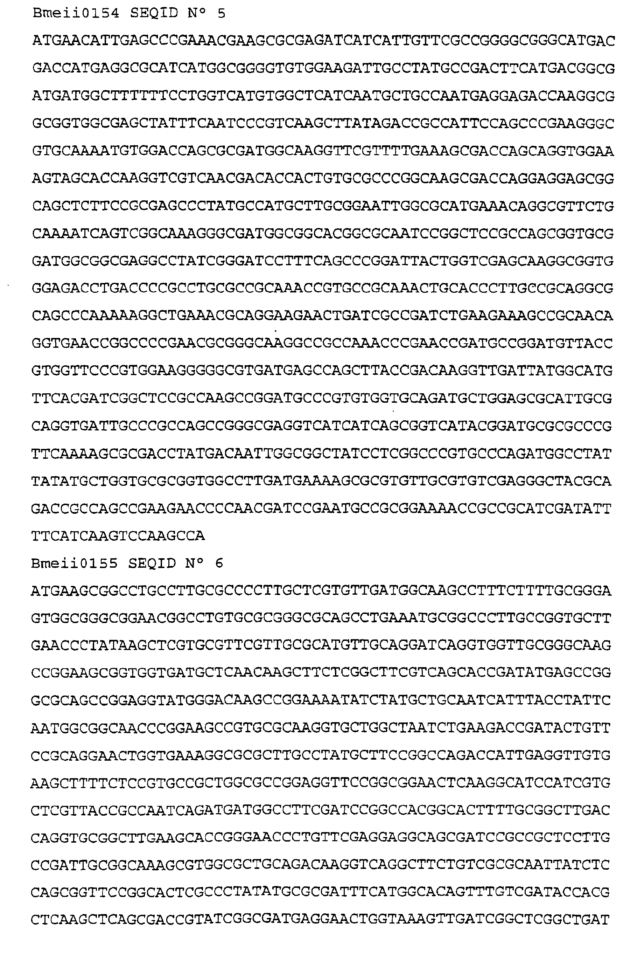

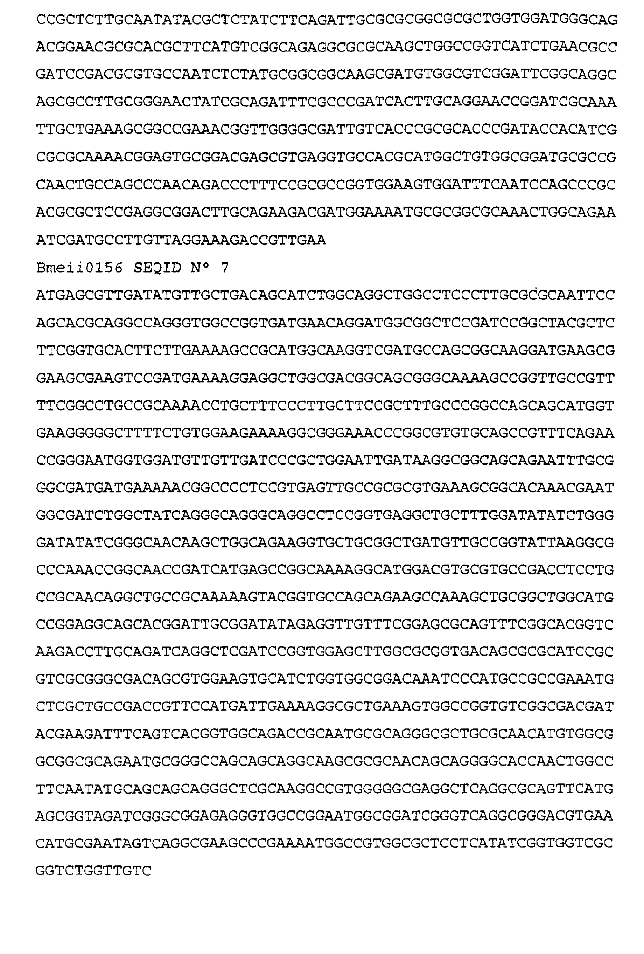

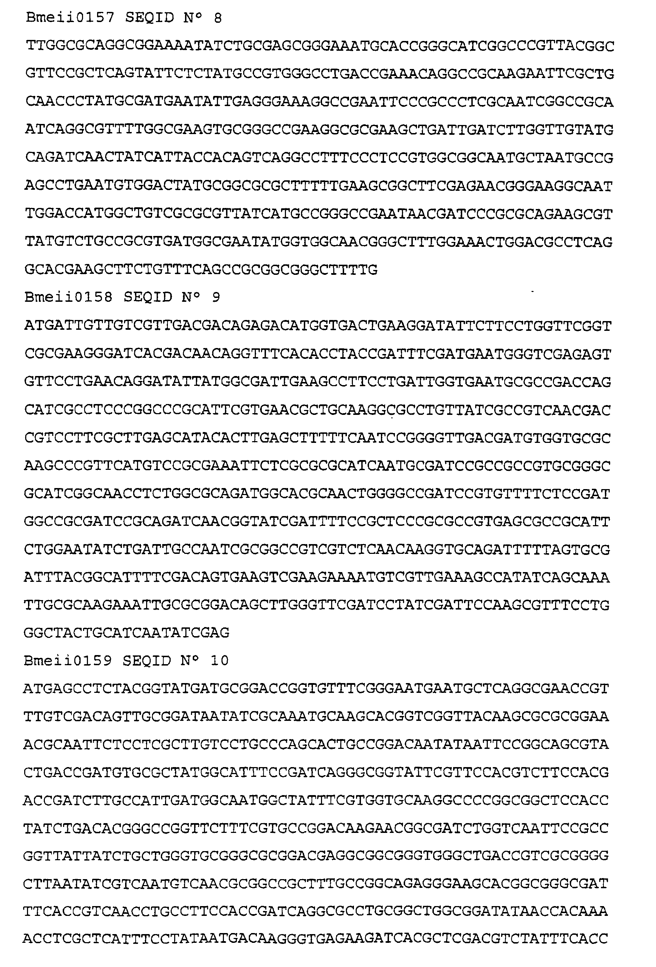

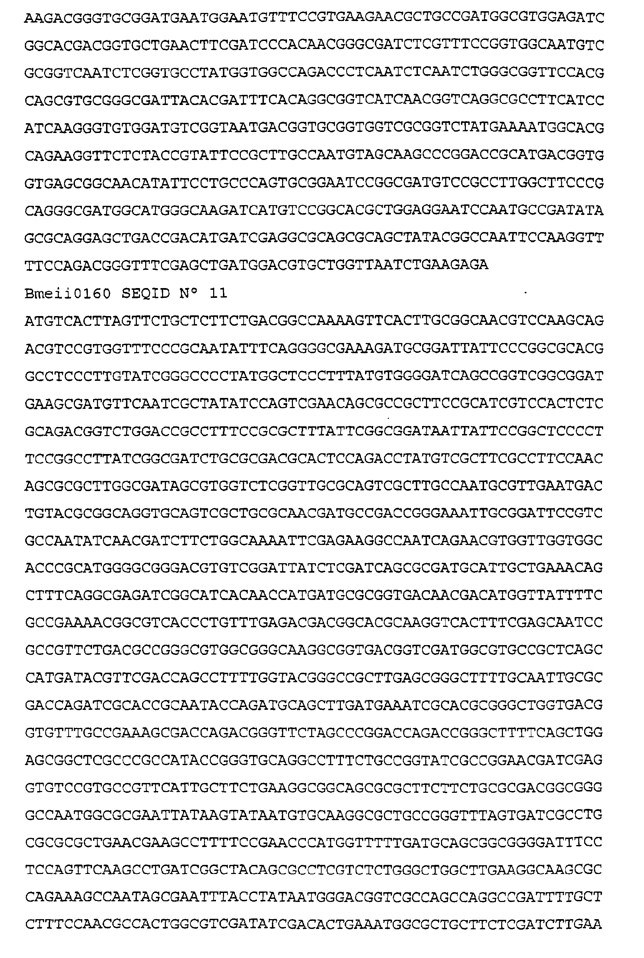

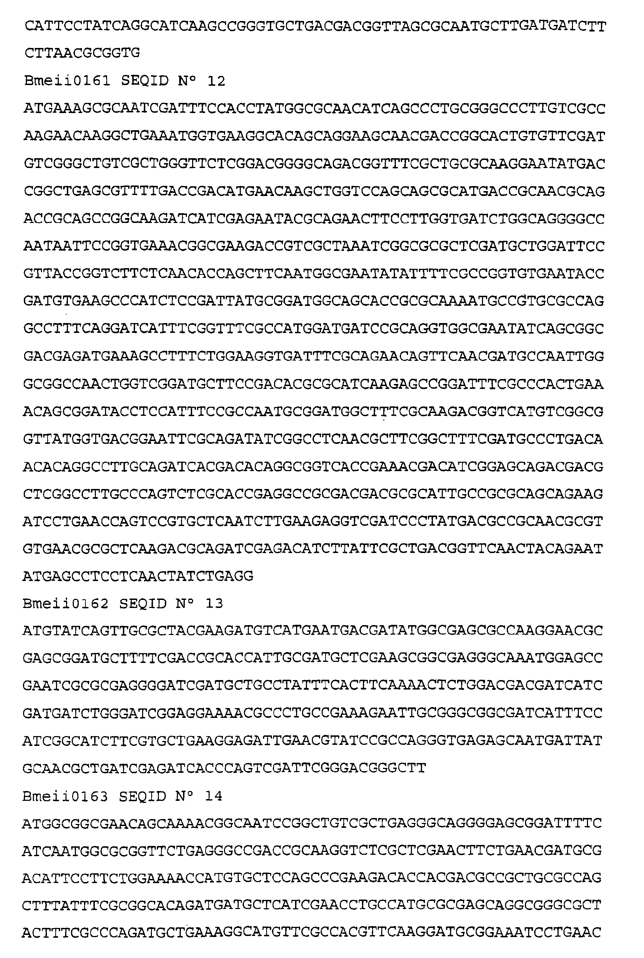

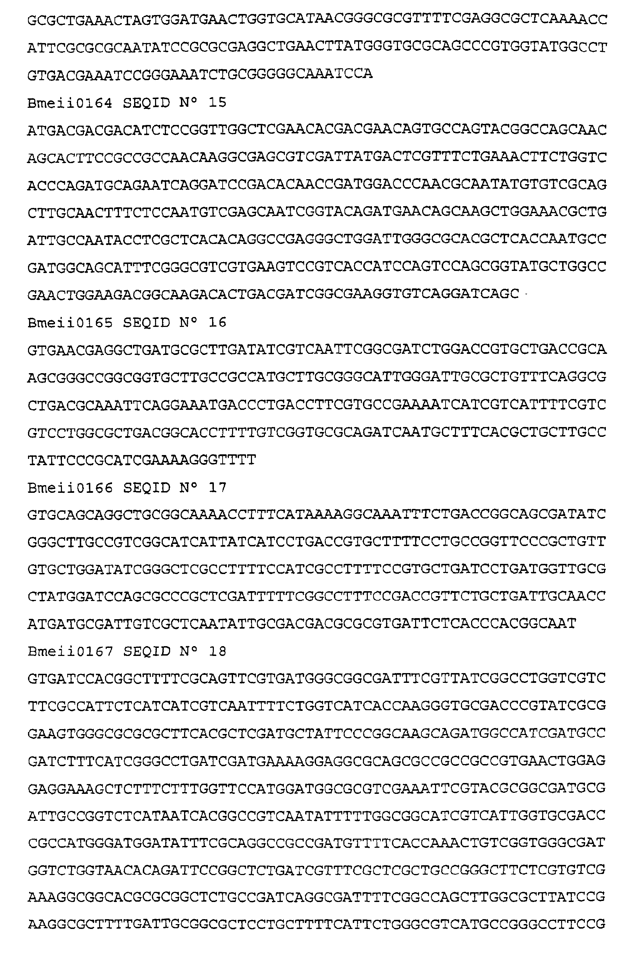

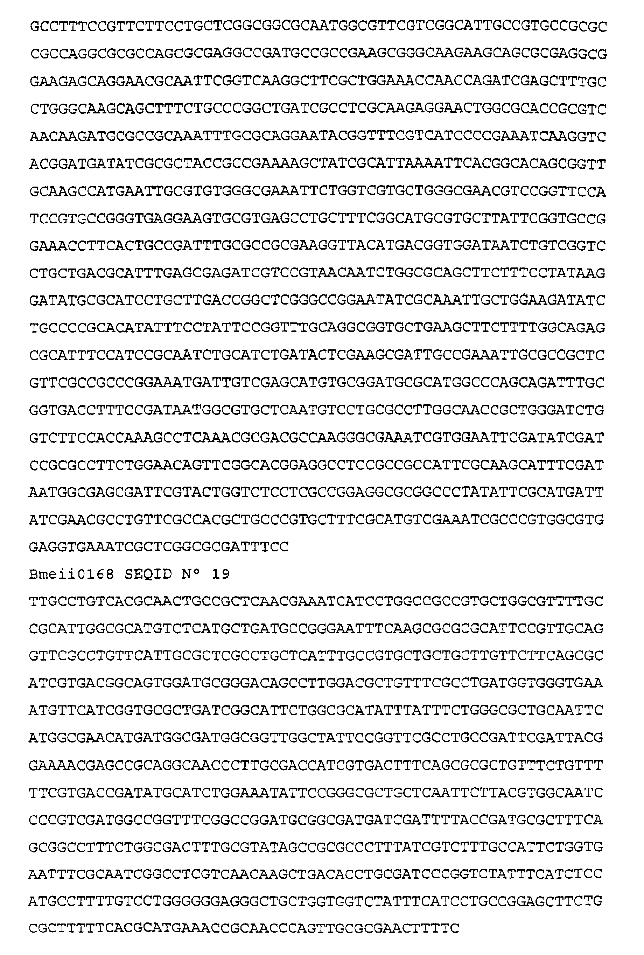

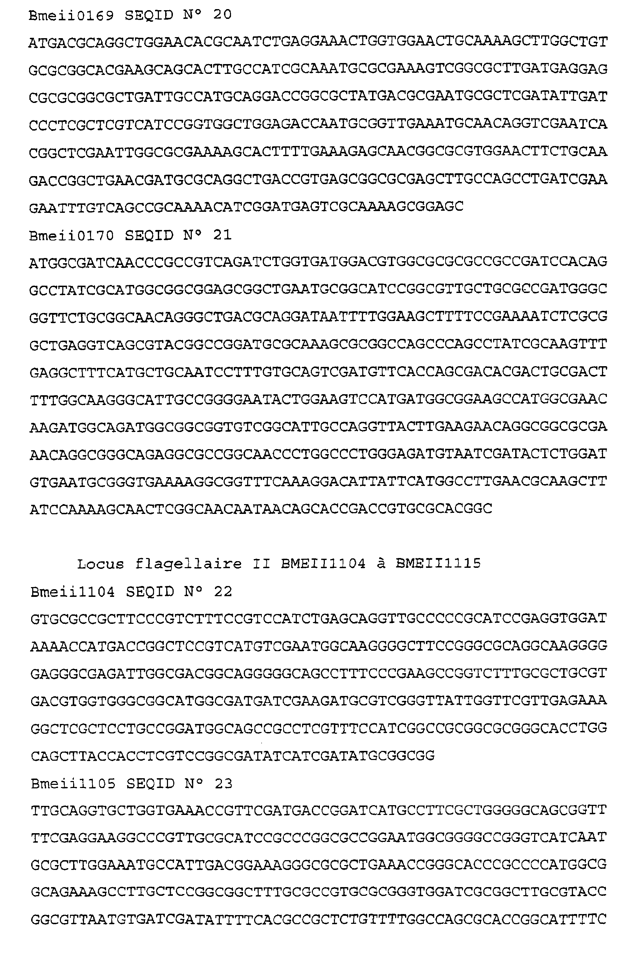

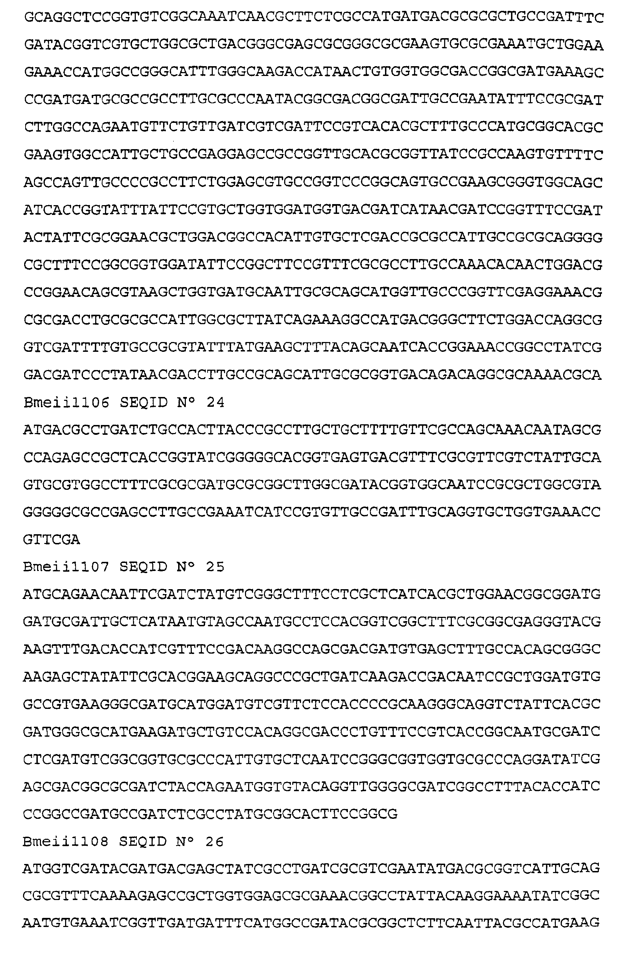

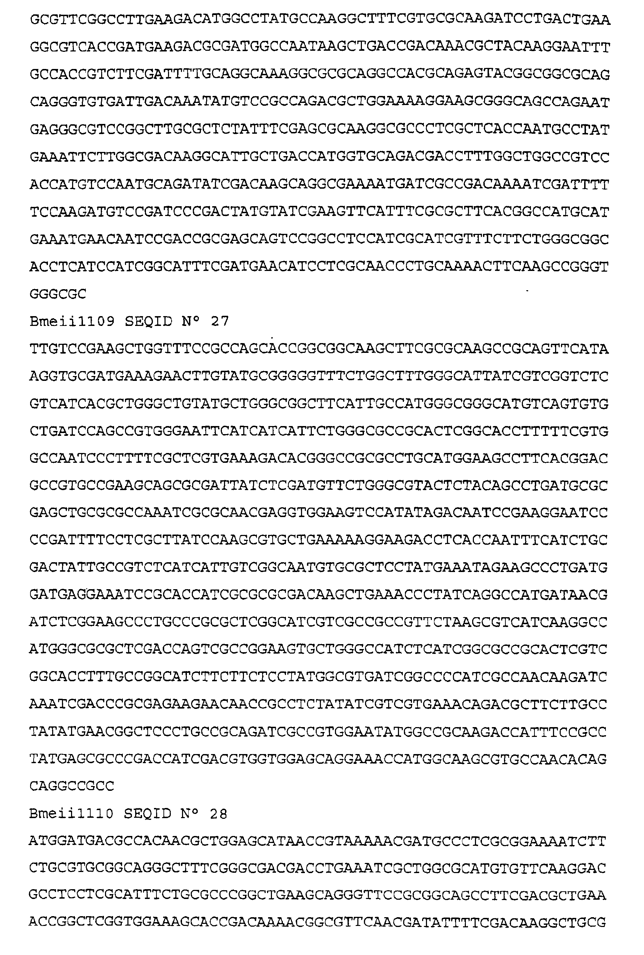

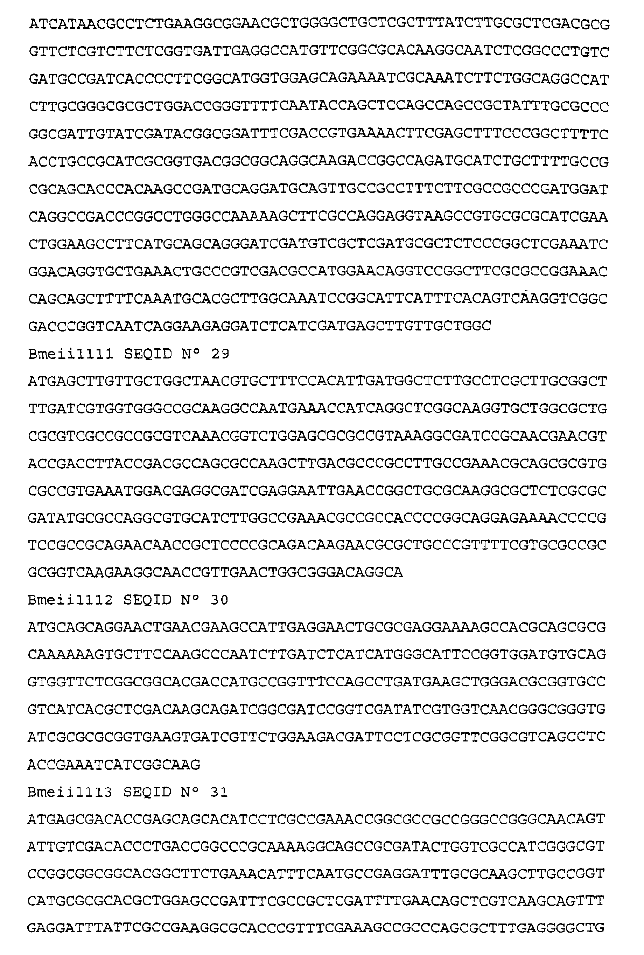

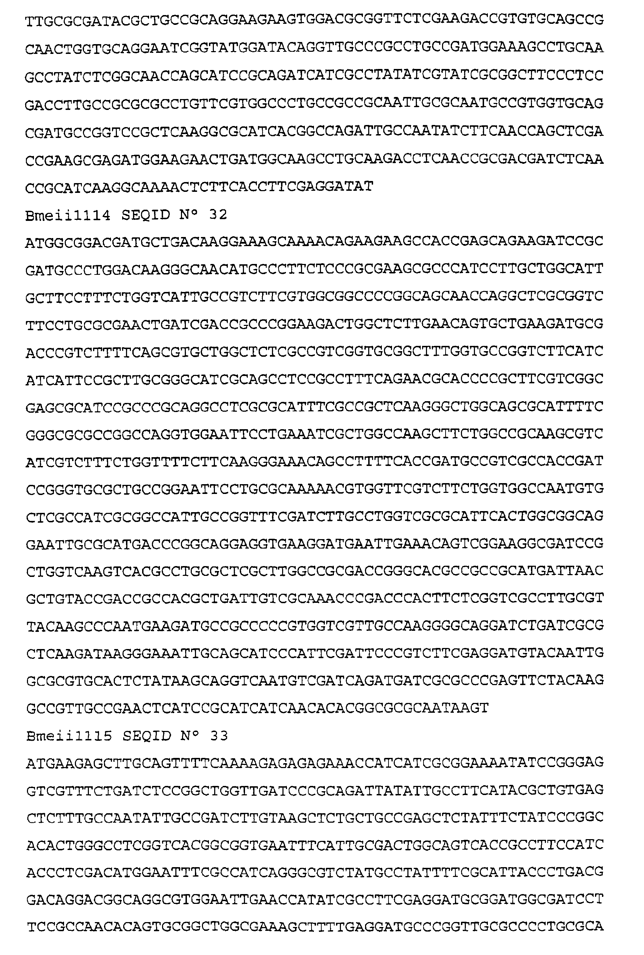

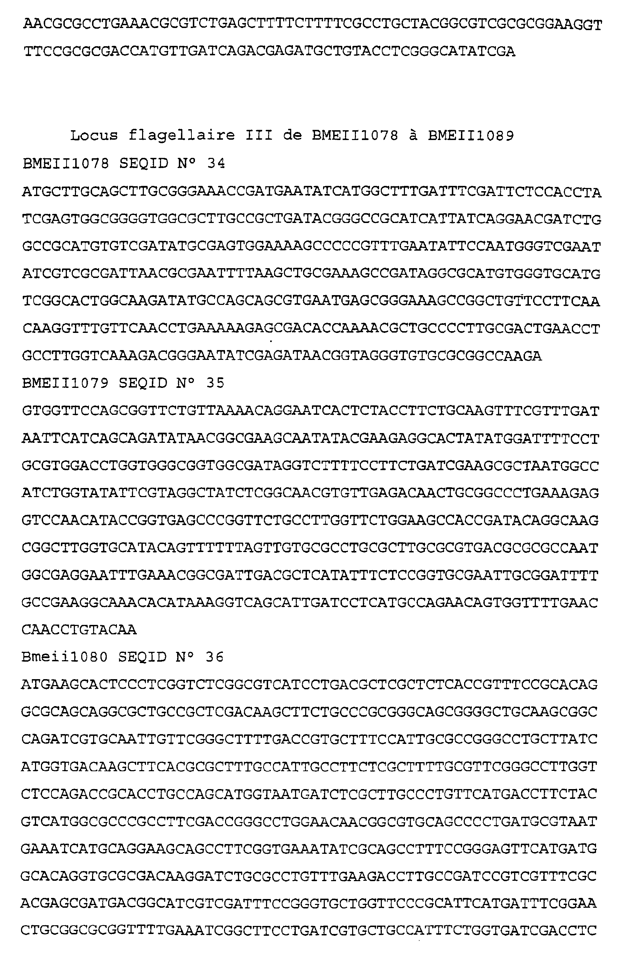

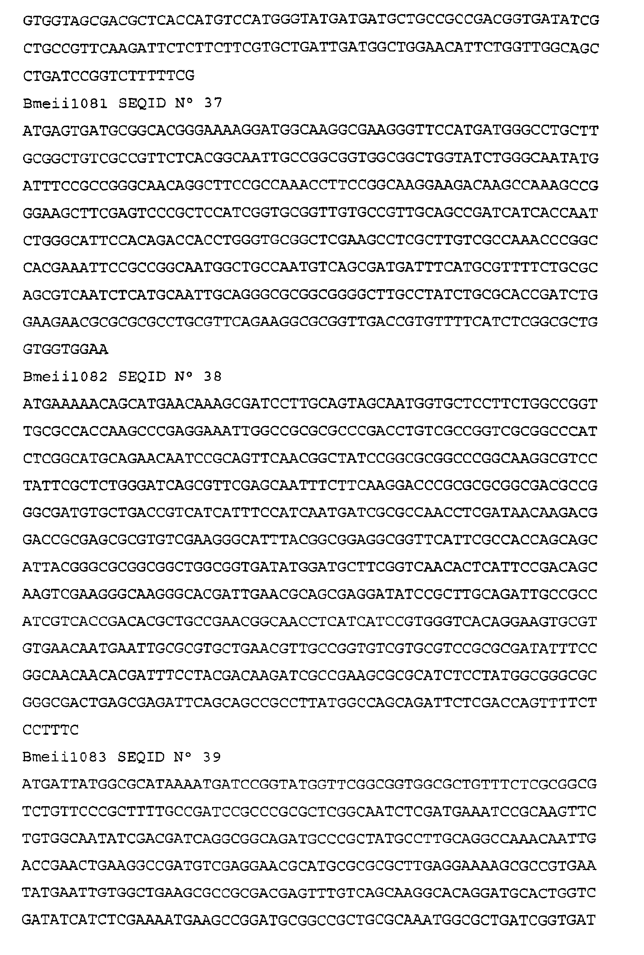

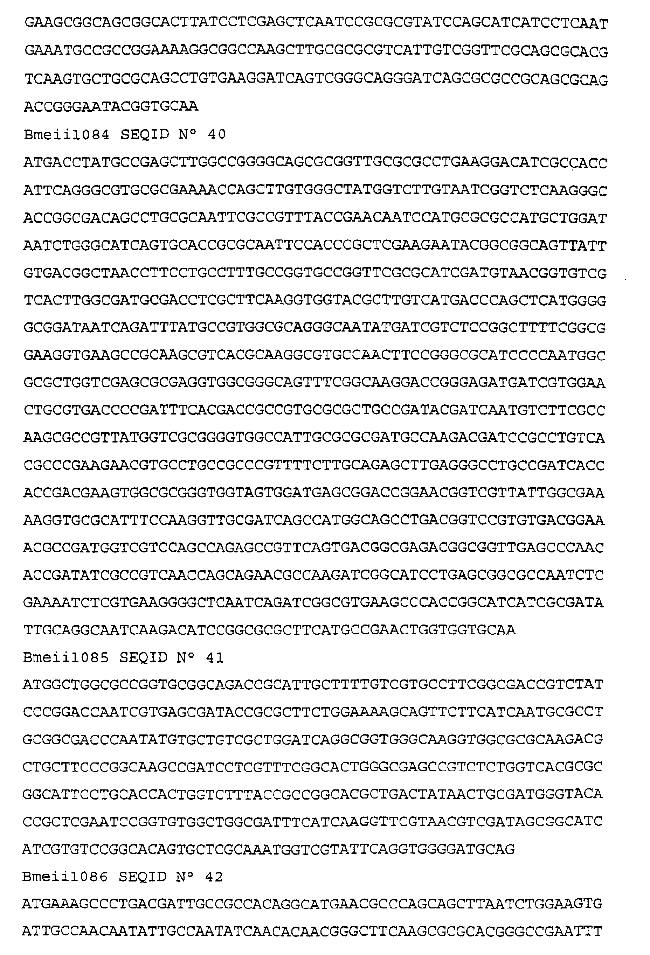

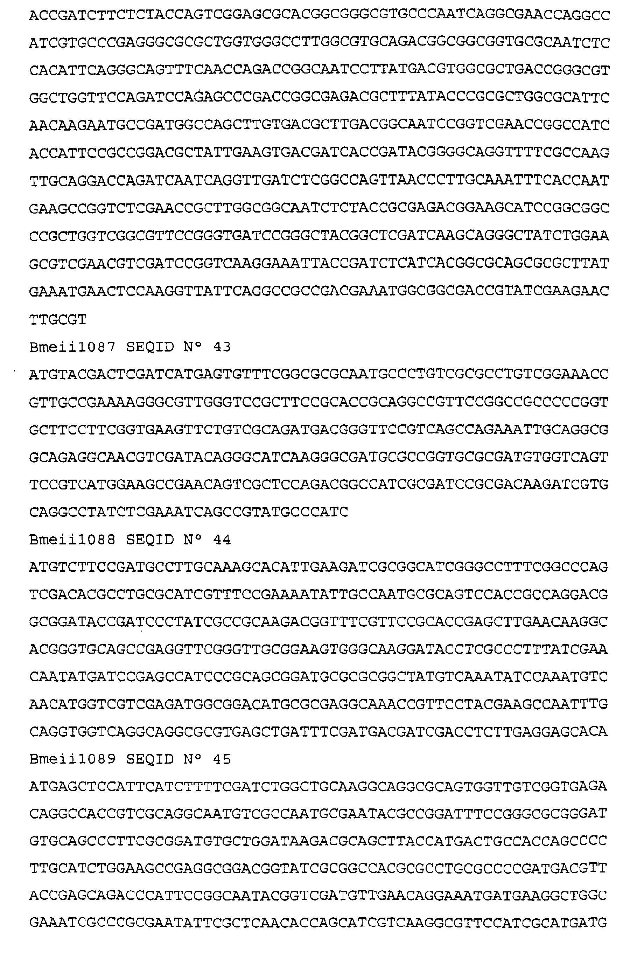

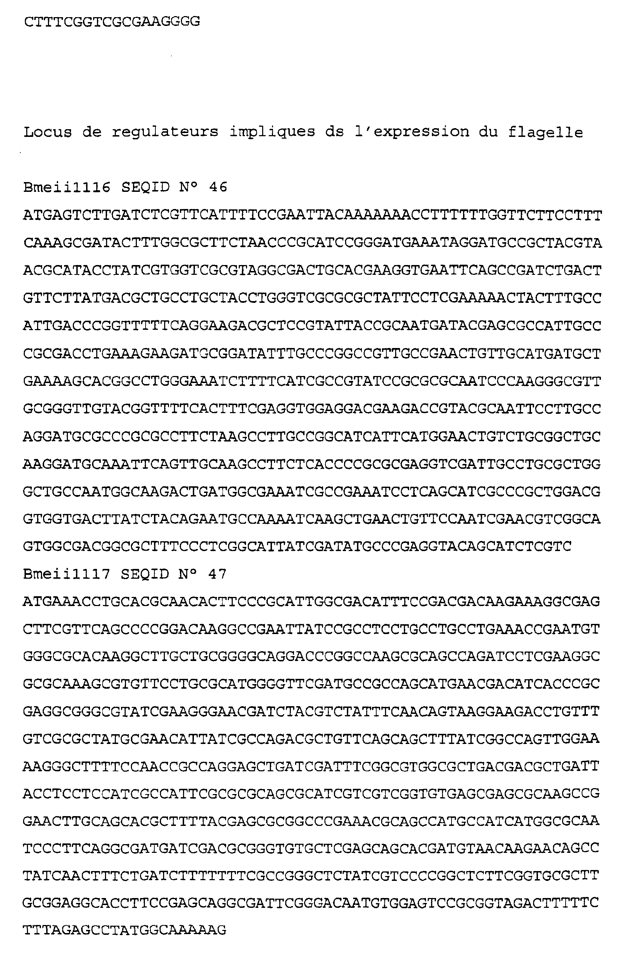

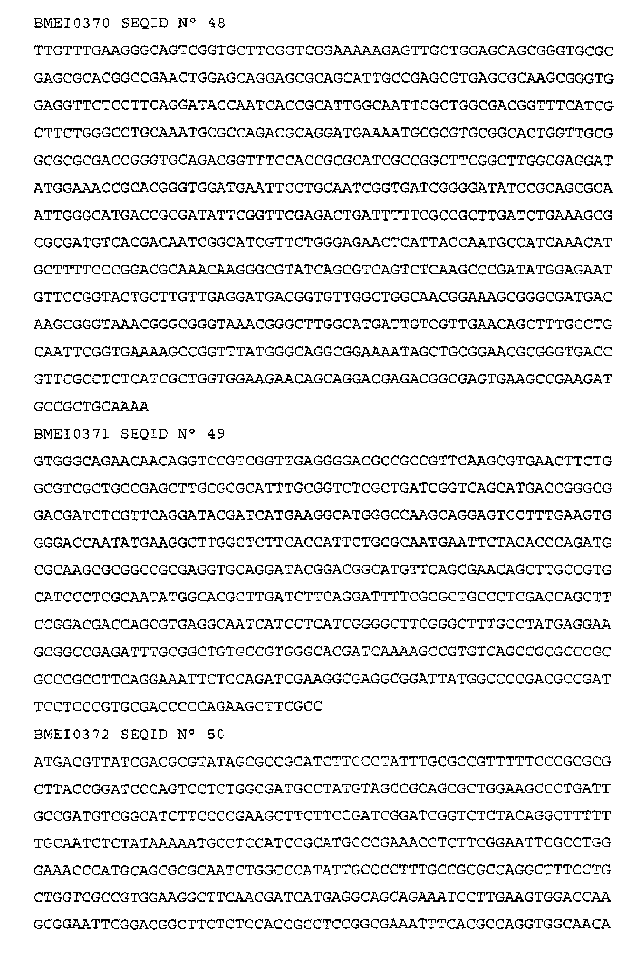

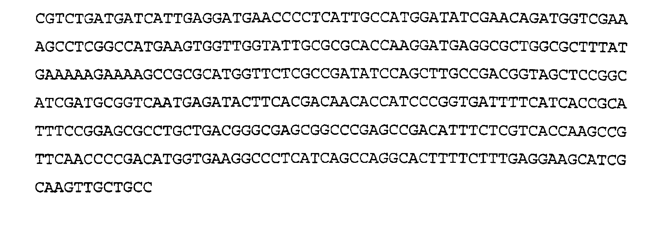

En particulier, l'objet de la présente invention concerne au moins une séquence choisie parmi le groupe constitué par les séquences SEQID N° 1 à SEQID N° 50 (BMEI0370 à 372, BMEII0150à 170, BMEII1078 à 1089, BMEII1104 à 1117) encodant des protéines flagellaires ou leur régulateurs présentes dans le génome de *Brucella*.(fig. 2).

Ces séquences représentent l'ensemble des protéines nécessaires à la biosynthèse de l'appareil de sécrétion des protéines flagellaires à l'ultrastructure flagellaire proprement dite et au moteur ainsi que des protéines impliquées dans la régulation de l'expression de ces protéines. Par contre, aucune séquence codante apparentée au système de chemotactisme n'a été identifiée parmi ces séquences.

De préférence, l'invention concerne les séquences impliquées dans les cycles infectieux chez la souris (en particulier les séquences *fliC*, *flgE*, *motB*, *flgI*, *flhA*, *2C2* et *fliF* identifiées respectivement par SEQID N°1, 10, 5, 40, 17, 18, 9 et 2.

La présente invention concerne également les portions et les variants de ces séquences ou de leurs brins complémentaires. Des portions de séquences selon l'invention concernent des séquences nucléotidiques ou peptidiques présentant les mêmes caractéristiques antigéniques et immunogéniques que la portion complète, ainsi que des portions susceptibles également de provoquer des cycles infectieux chez des mammifères, en particulier chez le modèle de la souris. Des variants de ces séquences englobent des séquences présentant une forte homologie ou une forte identité de séquences avec les séquences de l'invention.

De préférence, cette identité ou homologie de séquences est supérieure à 80%, de préférence supérieure à 85%, plus particulièrement supérieure à 95%, voire supérieure à 98 ou 99% avec la séquence nucléotidique ou peptidique complète choisie parmi le groupe constitué par les séquences SEQID N°1 à SEQID N° 50 (BMEI0370 à 372, BMEII0150 à 170, BMEII1078 à 1089, BMEII1104 à 1117).

L'objet de l'invention concerne également les protéines ou séquences polypeptidiques encodées par les séquences nucléotidiques susmentionnées, éventuellement modifiées ou liées par un ou plusieurs groupes de substitution, en particulier des groupes amides, acetyl, phosphoryl ou glycosil. De même, le polypeptide de l'invention peut être englobé dans une protéine plus large ou une protéine de fusion servant comme protéine porteuse du peptide de l'invention ou du fragment du peptide de l'invention. Des exemples de telles molécules porteuses sont par exemple la BSA, albumine bovine sérique ou l'hémocyanine.

Le polypeptide de l'invention peut également comporter un ou plusieurs acides aminés supplémentaires servant pour des séquences leaders ou des séquences importantes dans la sécrétion des peptides ou dans la purification des peptides, par exemple de multiples résidus histidine. De plus, les variants des séquences de l'invention peuvent comporter une ou plusieurs modifications nucléotidiques ou en acides aminés (de préférence de 1 à 10 acides aminés).

Les polypeptides de l'invention peuvent être présents sous plusieurs formes (cycliques, branchées ou à la fois branchées et cycliques), comporter la formation d'un ou de plusieurs ponts disulfure ou être soumis à différentes étapes de carboxylation, glycosylation, hydroxylation, iodination, méthylation, myristoylation, oxydation, telles que notamment décrites dans la référence "Proteins, structure and molecular properties", 2^{ème} éd. T.E. Creighton, New-York 1993, Wolt, F., "Posttranslational Protein Modifications : perspectives and prospects, p. 1 à 12 in Posttranslational covalent modification of proteins, B.C. Johnson éd. Académique presse New York 1983; Seifter et al., "Analysis for protein modifications and nonprotein cofactors", Methyl Enzymol. 1990, vol. 182, p. 626-646 et Rattan et al. "Protein synthesis : posttranslational modifications and aging" Ann. New York Acad. Sci. 1992, vol. 663, p. 48-62.

Les séquences polypeptidiques de l'invention concernent en particulier des séquences ADN ou ARN, simple brins ou double brins pouvant éventuellement comporter une ou plusieurs bases non usuelles, telles que l'inosine ou des bases marquées.

Les variants de l'invention peuvent être des variants naturels (présents éventuellement dans d'autres familles de souches ou dans d'autres espèces) ou des variants modifiés par les techniques de mutagènese ou par synthèse directe, mais conservant l'activité biologique du polypeptide de référence, en particulier ces propriétés antigéniques et immunogéniques. Les propriétés antigéniques pouvant être testées par des procédures standard (immunobloting) utilisant de préférence des sérums polyclonaux ou monoclonaux, en particulier dans les tests standard ELISA.

Le terme "identité de séquence" ou "homologie de séquence" sont des termes bien connus de l'homme de l'art (Carillo, H., and Lipton, D., SIAM J Applied Math 1998,vol. 48, p. 1073). Une identité de séquences peut être calculée par l'homme de l'art en utilisant des techniques bien connues (Computational molecular biology, Lesk, A.MK, éd. Oxfort University Press, New York 1988 ; Biocomputing : informatics and genome projects, Smith, D.W., éd. Academic Press, New York 1993; Computer Analysis of sequence data, part I,Griffin, A.M., and Griffin, H.G., Humana Press, New Jersey 1994; Sequence analysis in molecular biology, von Heijne, G., Academic Press 1987 and sequence analysis primer, Gribskov, M. and Devereux, J. éd. Stockton Press, New York 1991).

De même, des méthodes pour caractériser une identité ou une similarité entre deux séquences génétiques peuvent utiliser les programmes informatiques connus, tels que les programmes Blast et G.C.G.

Les portions ou fragments des séquences polypeptidiques et nucléotidiques de l'invention comprennent des séquences structurelles et fonctionnelles importantes, en particulier les séquences comprenant des hélices alpha ou des plans bêta, les régions formant des boucles, les régions hydrophiles ou les régions hydrophobes et les portions comprenant des index antigéniques épitopes ou linéaires ou conformationnels élevés.

Un autre aspect de la présente invention concerne également un vecteur comprenant une ou plusieurs séquences génétiques (nucléotidique et en acide aminé) de l'invention, ce vecteur pouvant être un élément susceptible d'obtenir la transfection ou la transduction d'une cellule, en particulier un plasmide, un virus, un liposome ou une vésicule cationique.

Un autre aspect de la présente invention concerne la cellule transfectée ou comprenant le vecteur de l'invention, ainsi qu'une souche de Brucella dépourvue d'une ou plusieurs des séquences génétiques de l'invention ou une souche de Brucella dont une ou plusieurs de ces séquences sont modifiées (mutées ou délétées) de manière à éliminer le caractère virulent de ces séquences.

L'objet de la présente invention également un inhibiteur dirigé contre les séquences génétiques de l'invention et/ou susceptible d'empêcher leur expression. Un tel inhibiteur peut être par exemple un anticorps ou une portion hyper variable de cet anticorps produit par une lignée cellulaire (hybridome exprimant cet inhibiteur). Un autre exemple inhibiteur peut être une molécule antisens ou un ribozyme susceptible de s'hybrider de manière complémentaire avec la séquence nucléotidique de l'invention (séquence codante ou séquence de régulation (promoteur/opérateur, etc.)) pour empêcher son expression ou effectuer son clivage.

Un autre aspect de la présente invention est relatif à une composition pharmaceutique comprenant un véhicule ou diluant pharmaceutique adéquat ainsi qu'un élément choisi parmi le groupe constitué par les séquences génétiques de l'invention (polynucléotide et polypeptide), leurs variants ou leurs portions, le vecteur de l'invention et éventuellement la cellule transformée ou l'inhibiteur de l'invention.

De manière avantageuse, la composition pharmaceutique de l'invention est un vaccin comportant éventuellement un ou plusieurs adjuvants susceptibles d'augmenter la réponse immunitaire d'un patient, en particulier d'un mammifère (ruminant) contre *Brucella*.

L'objet de l'invention concerne également un procédé de traitement et/ou de prévention d'une maladie susceptible d'affecter un mammifère (ruminant), en particulier un syndrome induit par les agents de *Brucella* chez ledit mammifère, en particulier les ruminants tels que les moutons (y compris chez l'homme) (maladie de Malte); ce procédé comprenant l'étape d'administration à ce patient d'une quantité suffisante de la composition pharmaceutique de l'invention, (éventuellement sous forme de vaccin), de manière à prévenir les syndromes induits par l'agent pathogène *Brucella* ainsi que la transmission de cet agent pathogène à d'autres mammifères.

Un autre aspect de l'invention concerne également une trousse de diagnostic pour déterminer une pathologie induite par l'agent pathogène (Brucella) et comprenant les séquences génétiques de l'invention, ou les inhibiteurs dirigés contre ces séquences génétiques.

Un dernier aspect de la présente invention concerne l'utilisation de la composition de l'invention pour la fabrication d'un médicament destiné à la prévention ou au traitement des infections induites par *Brucella* chez les mammifères (y compris chez l'homme).

La présente invention sera décrite en détails dans une forme d'exécution préférée en référence aux figures annexées.

### Brève description des figures

La figure 1 montre l'atténuation du mutant transpositionnel fliF chez la souris BALB/c.

La figure 2 représente les séquences de l'invention.

### Description détaillée de l'invention

A titre d'exemple, les inventeurs ont mis en évidence l'expression de trois séquences de l'invention : fliF (BMEII0151) ex vivo et de fliF (BMEII0151), flgE (BMEII0159) et fliC (BMEII0150) in vitro. L'expression de ces trois séquences qui représentent les différents niveaux de la structure flagellaire (les monomères de l'anneau MS, du coude et du filament) était coordonnée. Ces différentes données permettent de proposer l'existence d'un régulon flagellaire fonctionnel chez *Brucella.*

Le système flagellaire de *Brucella* est apparenté à celui d'autres rhizobiacés. Au sein de ce groupe, le parent le plus proche de *Brucella* est *Mesorhizobium loti.* Pour les CDS flagellaires, cette parenté est marquée à deux niveaux 1° l'homologie de séquence et 2° l'organisation de CDS au sein des loci.

Un scénario évolutif est proposé par Moreno : l'ancêtre de *Brucella* serait une bactérie flagellée mobile vivant dans le sol (Moreno *et al.*, 2002). Cette bactérie est devenue progressivement un pathogène intracellulaire et s'est adaptée au mode de vie parasitaire. Au niveau flagellaire, elle perd son système chemotactique. Cet auteur émet l'hypothèse que les séquences flagellaires soient en fait des « fossiles » issus de ces ancêtres mobiles. Cette hypothèse semble peu vraisemblable. Par rapport à son ancêtre, *Brucella* a réduit son génome et malgré cela l'inventaire révèle que 1% de son matériel génétique est dédié aux CDS flagellaires, il est donc probable qu'elles aient une fonction. Pour d'autres auteurs, la parenté entre les systèmes flagellaires et les systèmes de sécrétion de type III laisse supposer que *Brucella* a en fait reconverti ces CDS flagellaires en un système de sécrétion (DelVecchio *et al*., 2002) (Ugalde, 1999). L'inventaire des CDS que nous avons réalisé nous a amené à poser la question de l'existence transitoire d'un flagelle chez *Brucella*.

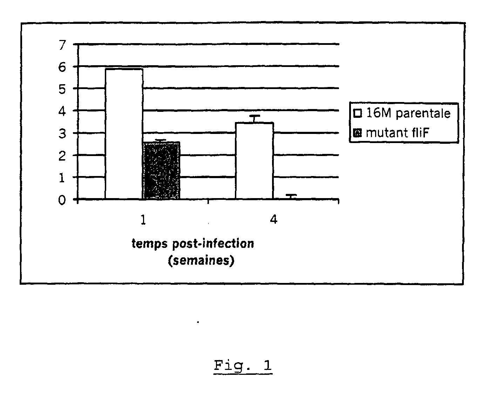

Un mutant dans le CDS *fliF* a été isolé lors d'un crible STM. Ce mutant est atténué chez la souris et dans les cellules phagocytaires professionnelles et non professionnelles (figure 1.). La virulence de la souche parentale B. melitensis 16M et du mutant transpositionnel fliF chez la souris BALB/c est obtenue par l'infection de la rate après injection intrapéritonéale de 10⁴ brucellae. (Les valeurs sont la moyenne plus ou moins l'écart type (n=4)). De même lors d'un crible d'expression différentielle de la GFP mené chez *B. suis* dans des macrophages murins, un clone possédant la région amont du CDS *fliF* a été isolé. Ce résultat a été confirmé chez *B. melitensis* dans deux autres types cellulaires (une lignée de macrophages bovins et des cellules HeLa). Ces premières données prouvent que le système flagellaire n'est pas cryptique et qu'il est requis pour le cycle infectieux. Par contre, à ce stade, l'hypothèse de la reconversion en un système de sécrétion de type III reste posée.

Cependant, d'autres CDS flagellaires sont requis pour le cycle infectieux de *B*. *melitensis*. Des mutants pour des CDS de l'ultrastructure flagellaire et du moteur (*fliC (BMEII0150)*, *flgE(BMEII0159)*, *flgI(BMEII1084)*, *motB*(*BMEII0154*) et pour des CDS du système de sécrétion (*flhA(BMEII0166-167)*, *flhB(BMEII1114)* ont été créés. Tous exceptée *flhB* sont requis pour le cycle infectieux chez la souris. Le mutant *flhB* semble n'être pas atténué parce que la stratégie expérimentale permet l'expression des domaines de la protéine ce qui entraînerait une complémentation intragénique. En conclusion, les CDS flagellaires ne sont pas exclusivement reconvertis en un système de sécrétion puisque des CDS encodant la flagelline le monomère du coude (FlgE) et l'anneau P (FlgI) sont impliqués dans le cycle infectieux. Un autre CDS flagellaire (FlgF, monomère de la tige) est également impliqué dans le cycle infectieux, il a été isolé lors d'un crible STM réalisé chez la chèvre.

Les difficultés techniques liées aux infections expérimentales ont amené les inventeurs à rechercher une condition d'expression *in vitro* des CDS flagellaires. Les inventeurs ont évalué l'expression des CDS flagellaires en fonction du stade d'une culture liquide et ont constaté l'expression de la flagelline, du monomère du coude et de l'anneau MS à faible densité de population. De plus, l'expression de ces CDS est concertée. Ces données amènent à conclure qu'il existe un régulon flagellaire chez *Brucella*. Des expériences en coloration négative de microscopie électronique sur *B*.*melitensis* cultivée en phase de latence ont permis d'observer des bactéries flagellées.

Ce résultat reste préliminaire. La faible concentration bactérienne au moment de l'expression des gène flagellaire et la fragilité de cette structure expliquent que cette expérience soit délicate. L'utilisation de mutants exprimant constitutivement un flagelle ou d'une autre condition d'expression devrait faciliter l'observation d'un flagelle et permettre de valider ce résultat.

Une des particularités de Brucella est, qu'elle est à ce jour, la seule bactérie qui exprime des CDS flagellaires et qui ne possède pas de système de chemotactisme. À long terme, il existe deux perspectives d'une part comprendre la fonction de ce système chez *Brucella* et d'autres part établir un schéma de la régulation de ce système, schéma qui pourra lui-même être intégré à celui d'autres systèmes impliqués dans la virulence.
**Mobilité :** la plus évidente fonction de ces gènes est de permettre à la bactérie de trouver son hôte. Chez *Brucella* l'absence de système chemotactique donne peu de poids à cette hypothèse. Néanmoins chez certaines bactéries la création de souches mutantes non chemotactiques conduit à une augmentation de la virulence dans des modèles d'infection. On peut donc supposer que *Brucella* ait perdu son système chemotactique mais qu'elle soit néanmoins mobile.
**Sécrétion :** chez *Yersinia enterolitica,* une phospholipase et probablement d'autres protéines impliquées dans la virulence sont sécrétées par l'appareil d'exportation des protéines flagellaires. Il est possible que *Brucella* sécrète des protéines impliquées dans la virulence. Il est difficile de comprendre l'intérêt pour la bactérie de synthétiser un filament connaissant le coût métabolique important pour la cellule. Une fonction sécrétrice serait une fonction accessoire que pourrait remplir les CDS flagellaires.

Différents critères peuvent être retenus pour sélectionner les effecteurs potentiellement secrétés :avoir une parenté avec un CDS eucaryotique, être à côté d'un petit gène pouvant servir de chaperonne, ne pas posséder une séquence signal. Une liste de 72 CDS présentant des homologies principalement avec des CDS eucaryotes a été établie. La plupart de ces CDS sont homologues à des CDS métaboliques et l'on peut difficilement leur prédire un rôle dans le cycle infectieux, il est difficile d'en sélectionner pour des expériences ultérieures.

Une deuxième approche expérimentale est donc plus recommandable. Des extraits protéiques peuvent êtres réalisés sur des surnageants de culture d'une *Brucella* sauvage et d'une souche disruptée pour l'appareil de sécrétion (le mutant *flif* ou *flhA* par exemple) et une approche protéomique permettra la comparaison des deux souches afin d'identifier ces éventuels candidats. D'autres fonctions peuvent être envisagées telle que l'adhésion ou bien des propriétés immunomodulatrices.

### Régulation de l'expression du système

**L'enclenchement de la cascade** : chez les bactéries pathogènes, la régulation de l'expression des gènes flagellaires est une étape importante dans la pathogénie. Certaines bactéries co-régulent leurs facteurs de virulence et leur flagelle (Akerley *et al.*, 1995). Les systèmes flagellaires sont très immunogènes et coûteux en énergie. La stratégie adoptée par certaines bactéries sera donc de réprimer leurs gènes flagellaires lors de l'entrée dans l'hôte tout en activant l'expression de facteur de virulence, c'est par exemple le cas pour *Bordetella*. Pour d'autres par contre ces systèmes seront absolument nécessaires pour accomplir le cycle de vie dans l'hôte. C'est par exemple le cas pour Legionella. Le gène 2C2 (BMEII0158) encode un régulateur des systèmes à deux composants. Dans ces systèmes, en réponse à un stimulus une histidine kinase s'autophosphoryle et transmet ce phosphate à un régulateur de réponse dont le rôle sera de moduler l'expression de certains gènes en réponse à ce stimulus. 2C2 encode un activateur de la transcription de l'opéron *fliF*. L'expression du 2C2. Pour comprendre cette régulation dans le cas du CDS 2C2 chez *Brucella*, il semble important dans un premier temps de muter l'aspartate 59 qui est le site de phosphorylation prédit. Deux types de mutations sont possibles : supprimer le site de phosphorylation en remplaçant l'aspartate par un glutamate et observer dans cette souche s'il y a. toujours transcription des CDS flagellaires ou bien mimer une phosphorylation constitutive en mutant l'aspartate en asparagine et également conserver l'effet sur les CDS flagellaires. On peut également envisager la production d'anticorps dirigé contre le 2C2 afin d'évaluer la présence de ce régulateur au cours d'une croissance en culture liquide car on ne peut exclure une régulation par protéolyse. La purification de la protéine et des expériences de protection à la DNAse sur le promoteur de *flif* permettraient d'identifier la séquence cible de ce régulateur, ce qui par la suite donnerait la possibilité d'une analyse bioinformatique du génome et ainsi de trouver d'autres CDS régulés par 2C2.

Dans les systèmes à deux composants décrits le régulateur est en général en opéron avec son histidine kinase. Le 2C2 n'est pas localisé à côté de son histidine kinase. Dans un second temps la recherche de l'histidine kinase est donc une étape clef pour comprendre la voie de transduction du signal qui conduit à l'activation de 2C2. 23 histidines kinases ont été prédites dans le génome. Il est donc intéressant de les muter ou bien de les utiliser dans un crible double hybride. Une fois cette kinase découverte, un schéma général de l'expression de ces deux protéines et de la phosphorylation peut être établi en fonction de croissance en culture liquide. Une fois ce schéma établi, il faut effectuer des liens entre les phénotypes observés (expression en phase de latence, en cellule) et les acteurs moléculaires identifiés et éventuellement établir un modèle de l'expression des CDS flagellaires. D'autres acteurs sont cerainement importants pour ce système de régulation. Chez *Sinorhizobium meliloti,* le CDS occupant la place du 2C2 dans le locus flagellaire est impliqué dans la régulation des CDS flagellaires (R.Shmitt, communication personnelle). Ce CDS est lui-même régulé par VisN/VisR. Chez Brucella à la place de ce dernier locus, on retrouve les CDS VjbR (BMEII1116) et TetR (BMEII1117). VjbR est également nécessaire à l'induction du promoteur de fliF et à l'expression de FlgE et de FliC. TetR semble avoir un effet sur l'activation du promoteur de fliF mais cela nécessite confirmation. Parmi les facteurs sigma de Brucella, celui encodé par l'ORF BMEI0371 est également impliqué dans la régulation de l'expression de FlgE et de FliC. La délétion de cette ORF entraîne en effet une surexpression de ces deux protéines. On émet l'hypothèse que ce facteur sigma régulerait un répresseur flagellaire. Le stade de la cascade falgellaire auquel ce facteur sigma intervient reste à déterminer. Cette ORF est entourée d'une histidine kinase (BMEII0370) et d'un régulateur de réponse de systèmes à deux composants (BMEII0372) dont le rôle dans l'expression des gènes flagellaires doit être testé.
Étant donné l'expression à faible densité de population, un effet répresseur d'un système de type quorum sensing peut être envisagé. Des tests en milieu conditionné (c'est-à-dire du milieu dans lequel on a déjà fait croître des bactéries) ou du milieu contenant les phéromones isolées de culture de Brucella permettraient de mettre en évidence un lien entre le quorum sensing et le système flagellaire.

Chez *Caulobacter cresentus,* l'expression du régulon flagellaire est dépendante du régulateur CtrA (Bellefontaine *et al.*, 2002). Un homologue fonctionnel de ce régulateur est présent chez *Brucella*.sp. Néanmoins il est peu probable qu'il soit impliqué dans le système flagellaire puisque la séquence consensus de liaison (TTAAN7TTAA) du régulateur a été identifiée et qu'elle ne se retrouve devant aucun CDS flagellaire.
**Le contrôle interne de l'expression.** Au cours de ce travail les inventeurs ont mis en évidence que l'expression de trois CDS est concertée. Sur base de l'analyse génomique, 13 unités de transcription dans les locus flagellaires sont prédites. Cette prédiction est à vérifier expérimentalement, soit par RT-PCR ou par des systèmes rapporteurs. Des tests de complémentation de ces mutants sont effectués pour détecter d'éventuels effets polaires sur les gènes situés en aval de la mutation de délétion. Les mutants fliF (BMEII0151) et 2C2 (BMEII0158) ont été complémentés par une copie plasmidique sauvage des gènes correspondants exprimés sous le contrôle de leur propre promoteur.

Les inventeurs ont également identifié lors de cet inventaire un homologue de *flbT (BMEII0163)*. Chez *Caulobacter crescentus*, FlbT est un répresseur traductionel qui contrôle l'expression de la flagelline. Si le CDS de *Brucella* est un homologue fonctionnel de FlbT, une souche délétée de ce gène devrait exprimer de la flagelline de manière constitutive.

Sur base des considérations développées ci-dessus et considérant les données préliminaires concernant l'atténuation de la virulence de mutants flagellaires dans le modèle souris, il est intéressant de valider à plus large échelle et sur une cinétique plus étendue leur atténuation en souris et par la même occasion d'examiner leur potentialité comme souche vaccinale protectrice à l'encontre d'un challenge infectieux.

L'utilisation initiale du modèle murin de protection permettra d'analyser plus de conditions avant d'éventuellement sélectionner certains mutants qui seront analysés par la suite dans un modèle d'infection faisant appel à l'espèce hôte : le mouton.

### Exemples :

### Construction du mutant flgI de B. melitensis

De manière à obtenir des vaccins atténués vivants d'une souche de *Brucella* atténuée.
séquence codante : le gène flgI encodant un homologue du monomère du P ring du flagelle, anneau localisé dans la membrane externe et faisant partie du corps basal d'une structure flagellaire, numéro de séquence codante dans le génome de B. melitensis 16M : BMEII1084. Séquence codante de 1074 nucléotides qui encode une protéine prédite de 358 résidus. La souche mutée porte deux copies incomplètes de flgI

### Origine de l'ADN et caractérisation

Amplification par PCR (réaction de polymérisation en chaîne) sur l'ADN génomique isolé du donneur 1 (B. melitensis 16M) avec les amorces oligonucléotidiques upflgI (5'-GCGCGCCTGAAGGACATC-3') et lowflgI (5'-ACGGCGGTCGTGAAATCG-3') d'un fragment allant du nucléotide 113 au nucléotide 635 de la phase codante *flgI* et nommé ici amplicon flgI
L'amplicon flgI d'une taille de 533 paires de bases a été cloné au site EcoRV du vecteur pSKKan, générant le vecteur pSKKan-flgI Le clonage de l'insert a été vérifié par séquençage. Le pSKKan a été obtenu par clonage du gène conférant la résistance à la kanamycine (amplifié par PCR à partir du plasmide pUC4K de Pharmacia avec les amorces kanamont (5'-GGGCATGCGGAAAGCCACGTTGTGTCT-3') et kanaval (5'-GGGGTGACCTTAGAAAAACTCATCGAGCAT-3') ) au site ScaI du gène *bla* du pSK-oriT. Le pSK-oriT est un dérivé du pBluescriptSK(-) (Stratagene) décrit dans Tibor et al, 2002, Infect and Immun, 70:5540-6. L'origine de réplication f1 du pBluescript a été excisée par restriction SspI et remplacée par un fragment de 0,76 kb SmaI-SalI contenant l'origine de transfert RK2.

### - obtention et description du mutant

Le vecteur pSKKAN contenant l'insert fragment interne de flgI (nommé pSKKan-flgI) a été transféré d'Escherichia coli S17-1 à B. melitensis 16M NalR par conjugaison. Le vecteur est intégré dans le génome de Brucella, dans le gène flgI, conduisant à deux copies incomplètes de ce gène. Les candidats intégrants sont résistants à la kanamycine. L'intégration site-spécifique a été vérifiée par southern blot sur de l'ADN génomique de clones candidats intégrants restreints par HindIII avec une sonde correspondant au gène conférant la résistance à la kanamycine.
La modification connue des propriétés de l'hôte, B. melitensis 16M est une atténuation dans le modèle murin BALB/c. Sa modification prévisible est son incapacité à exprimer les protéines flagellaires faisant partie des structures les plus externes (notamment la flagelline) et son incapacité à construire une structure flagellaire : on s'attend à une bactérie non-flagellée.

### Tests sur des modèles cellulaires in vitro

### Protocole

La survie des souches de *Brucella* a été évaluée sur des lignées de cellules immortalisées issues de macrophages d'origine péritonéale bovines (Stabel et Stabel, 1995) et dans des cellules cancéreuses HeLa par la procédure décrite par Delrue et *al*. (Delrue et *al*., 2001). Brièvement, les souches de *Brucella* sont mises en croissance en phase logarithmique pour 18 h en 2YT en présence d'antibiotiques appropriés et additionnées aux cellules à des taux d'infection de 200 à 300 par milieu de culture cellulaire. Les plaques de culture sont centrifugées pendant 5 minutes pour les macrophages et pendant 10 minutes pour les cellules HeLa à 1000 tours par minute à température ambiante et placées en atmosphère 5% CO₂ à 37°C. Après une heure, les plaques sont lavées et incubées avec un milieu de culture supplémentaire comprenant 50µg de gentamicin (Life Technologies) par ml pour tuer toutes les cellules de *Brucella* extra-cellulaires. Le nombre de souches de *Brucella* intra-cellulaires viables est déterminé après deux heures ou 48 h de post-infection. Les données sont la moyenne de trois milieux de culture et représentent trois expériences.

Les mutants flagellaires flgI de *B. melitensis* ne montrent aucune atténuation significative dans ces modèles d'infection de lignées cellulaires.

### Protocole

Essai n° 1 : des souris femelles de huit semaines sont inoculées de manière intra-péritonéale avec 0,1 ml d'une suspension comprenant 5 x 10⁴ CFU de chaque souche bactérienne (mutant flgI et 16M NalR de souche parente) cultivée en présence de PBS (chaque dose étant confirmée rétrospectivement). Après 8 et 12 semaines d'inoculation, 5 souris de chaque groupe de traitement sont sacrifiées. Le taux de survie des bactéries est déterminé après homogénisation des rates des souris dans 2ml d'eau distillée. Des dilutions en série des homogénats sont mises en culture sur TSA pour déterminer les concentrations bactériennes.

| Essai 1 : en moyenne CFU par rate : n=5 (SD) | | |
|---|---|---|
| souche | 8 w pi | 12 w pi |
| mutant FlgI | 74,7(167) | 8(20) |
| parent16M nalR | 728(817) | 143(288) |

| Essai 2 : moyenne logarithmique CFU par rate n=4 (SD) | | | | |
|---|---|---|---|---|
| souche | 1 w pi | 4 w pi | 8 w pi | 12 w pi |
| parent16M NalR | 5,6 (1,1 ; n=3) | 5,41 (0,61) | 4,2 (0,5) | |
| mutantFlgI t | 5,89 (0,16) | 2,83 (0,5) | 1,32 (1,58 ; 0 cfu pour n=2) | |

### Tests de protection sur souris

### Protocole

Une femelle de huit semaines a été inoculée de manière intra-péritonéale avec 0,1 ml d'une suspension contenant environ 5 x 10⁴ CFU du mutant flgI mutant cultivé en présence de PBS (les doses exactes ont été rétrospectivement confirmées) (PBS était utilisé comme contrôle négatif. A huit semaines, après inoculation, 4 souris de chaque traitement mise en présence de *B.* *melitensis* 16M par inoculation intra-péritonéale de 1 x 10⁴ CFU ont été sacrifiées quatre semaines après pour collecter les rates (12 semaines de post-vaccination). Le taux de survie des bactéries a été déterminé après homogénisation des rates de souris dans 2 ml d'eau distillée. Les dilutions en série des homogénats ont été mises en culture sur TSA et sur TSA contenant la kanamycine pour déterminer la concentration de bactéries de la souche parente (sensible à la kanamycine) et de la souche mutée (résistante à la kanamycine).

| souche vaccinale | souche vaccinale CFU | moyenneCFU souche 16M (SD)protection | protéction |
|---|---|---|---|
| FlgI | 0 | 0 | Total |
| PBS | 0 | 1,0 x 10⁵ (1,5 x 10⁵) | |

### Tests sur brebis gestantes

### Informations sur le croisement et le transfert génétique

### Insert 1.1 Origine sub-cellulaire de l'ADN.

Amplification par PCR (réaction de polymérisation en chaîne) sur l'ADN génomique isolé du donneur 1 avec les amorces oligonucléotidiques upflgI (5'-GCGCGCCTGAAGGACATC-3') et lowflgI (5'-ACGGCGGTCGTGAAATCG-3') d'un fragment allant du nucléotide 113 au nucléotide 635 de la phase codante *flgI* et nommé ici amplicon flgI.

L'amplicon flgI d'une taille de 533 paires de bases a été cloné au site EcoRV du vecteur pSKKan, générant le vecteur pSKKan-flgI. Le pSKKan a été obtenu par clonage du gène conférant la résistance à la kanamycine (amplifié par PCR à partir du plasmide pUC4K de Pharmacia avec les amorces kanamont (5'-GGGCATGCGGAAAGCCACGTTGTGTCT-3') and kanaval (5'-GGGGTGACCTTAGAAAAACTCATCGAGCAT-3') ) au site ScaI du gène bla du pSK-oriT. Le pSK-oriT est un dérivé du pBluescriptSK(-) (Stratagene) décrit dans Tibor et al, 2002 (Infect and Immun, 70:5540-6). L'origine de réplication f1 du pBluescript a été exciseéé par restriction SspI et remplacée par un fragment de 0,76 kb SmaI-SalI contenant l'origine de transfert RK2.

La séquence de l'amplicon flgI est la suivante :

GCGCGCCTGAAGGACATCGCCACC
ATTCAGGGCGTGCGCGAAAACCAGCTTGTGGCTATGGTCTTGTAATCGGTCTCAAGGG
CACCGGCGACAGCCTGCGCAATTCGCCGTTTACCGAACAATCCATGCGCGCCATGCTGG
ATAATCTGGGCATCAGTGCACCGCGCAATTCCACCCGCTCGAAGAATACGGCGGCAGTT
ATTGTGACGGCTAACCTTCCTGCCTTTGCCGGTGCCGGTTCGCGCATCGATGTAACGGT
GTCGTCACTTGGCGATGCGACCTCGCTTCAAGGTGGTACGCTTGTCATGACCCAGCTCA
TGGGGGCGGATAATCAGATTTATGCCGTGGCGCAGGGCAATATGATCGTCTCCGGCTTT
TCGGCGGAAGGTGAAGCCGCAAGCGTCACGCAAGGCGTGCCAACTTCCGGGCGCATCCC
CAATGGCGCGCTGGTCGAGCGCGAGGTGGCGGGCAGTTTCGGCAAGGACCGGGAGATGA
TCGTGGAACTGCGTGACCCCGATTTCACGACCGCCGT

L'amplicon flgI, encodant un homologue du monomère du P ring du flagelle, anneau localisé dans la membrane externe et faisant partie du corps basal d'une structure flagellaire (numéro de séquence codante dans le génome de B. melitensis 16M : BMEII1084). Cette séquence codante de 1074 nucléotides encode une protéine prédite de 358 résidus. La souche mutée porte deux copies incomplètes de flgI :

séquence de flgI :
ATGACCTATGCCGAGCTTGGCCGGGCAGCGCGGTTGCGCGCCTGAAGGACATCGCCAC
C
ATTCAGGGCGTGCGCGAAAACCAGCTTGTGGGCTATGGTCTTGTAATCGGTCTCAAGGG

CACCGGCGACAGCCTGCGCAATTCGCCGTTTACCGAACAATCCATGCGCGCCATGCTGG
ATAATCTGGGCATCAGTGCACCGCGCAATTCCACCCGCTCGAAGAATACGGCGGCAGTT
ATTGTGACGGCTAACCTTCCTGCCTTTGCCGGTGCCGGTTCGCGCATCGATGTAACGGT
GTCGTCACTTGGCGATGCGACCTCGCTTCAAGGTGGTACGCTTGTCATGACCCAGCTCA
TGGGGGCGGATAATCAGATTTATGCCGTGGCGCAGGGCAATATGATCGTCTCCGGCTTT
TCGGCGGAAGGTGAAGCCGCAAGCGTCACGCAAGGCGTGCCAACTTCCGGGCGCATCCC
CAATGGCGCGCTGGTCGAGCGCGAGGTGGCGGGCAGTTTCGGCAAGGACCGGGAGATGA
TCGTGGAACTGCGTGACCCCGATTTCACGACCGCCGTGCGCGCTGCCGATACGATCAAT
GTCTTCGCCAAGCGCCGTTATGGTCGCGGGGTGGCCATTGCGCGCGATGCCAAGACGAT
CCGCCTGTCACGCCCGAAGAACGTGCCTGCCGCCCGTTTTCTTGCAGAGCTTGAGGCC
TGCCGATCACCACCGACGAAGTGGCGCGGGTGGTAGTGGATGAGCGGACCGGAACGGTC
GTTATTGGCGAAAAGGTGCGCATTTCCAAGGTTGCGATCAGCCATGGCAGCCTGACGGT
CCGTGTGACGGAAACGCCGATGGTCGTCCAGCCAGAGCCGTTCAGTGACGGCGAGACGG
CGGTTGAGCCCAACACCGATATCGCCGTCAACCAGCAGAACGCCAAGATCGGCATCCTG
AGCGGCGCCAATCTCGAAAATCTCGTGAAGGGGCTCAATCAGATCGGCGTGAAGCCCAC
CGGCATCATCGCGATATTGCAGGCAATCAAGACATCCGGCGCGCTTCATGCCGAACTGG
TGGTGCAA

La séquence des deux copies incomplètes est la suivante :

ATGACCTATGCCGAGCTTGGCCGGGGCAGCGCGGTTGCGCGCCTGAAGGACATCGCCAC
C
ATTCAGGGCGTGCGCGAAAACCAGCTTGTGGCTATGGTCTTGTAATCGGTCTCAAGGG
CACCGGCGACAGCCTGCGCAATTCGCCGTTTACCGAACAATCCATGCGCGCCATGCTGG
ATAATCTGGGCATCAGTGCACCGCGCAATTCCACCCGCTCGAAGAATACGGCGGCAGTT
ATTGTGACGGCTAACCTTCCTGCCTTTGCCGGTGCCGGTTCGCGCATCGATGTAACGGT
GTCGTCACTTGGCGATGCGACCTCGCTTCAAGGTGGTACGCTTGTCATGACCCAGCTCA
TGGGGGCGGATAATCAGATTTATGCCGTGGCGCAGGGCAATATGATCGTCTCCGGCTTT
TCGGCGGAAGGTGAAGCCGCAAGCGTCACGCAAGGCGTGCCAACTTCCGGGCGCATCCC
CAATGGCGCGCTGGTCGAGCGCGAGGTGGCGGGCAGTTTCGGCAAGGACCGGGAGATGA
TCGTGGAACTGCGTGACCCCGATTTCACGACCGCCGT

GCGCGCCTGAAGGACATCGCCACC
ATTCAGGGCGTGCGCGAAAACCAGCTTGTGGCTATGGTCTTGTAATCGGTCTCAAGGG
CACCGGCGACAGCCTGCGCAATTCGCCGTTTACCGAACAATCCATGCGCGCCATGCTGG

ATAATCTGGGCATCAGTGCACCGCGCAATTCCACCCGCTCGAAGAATACGGCGGCAGTT
ATTGTGACGGCTAACCTTCCTGCCTTTGCCGGTGCCGGTTCGCGCATCGATGTAACGGT
GTCGTCACTTGGCGATGCGACCTCGCTTCAAGGTGGTACGCTTGTCATGACCCAGCTCA
TGGGGGCGGATAATCAGATTTATGCCGTGGCGCAGGGCAATATGATCGTCTCCGGCTTT
TCGGCGGAAGGTGAAGCCGCAAGCGTCACGCAAGGCGTGCCAACTTCCGGGCGCATCCC
CAATGGCGCGCTGGTCGAGCGCGAGGTGGCGGGCAGTTTCGGCAAGGACCGGGAGATGA
TCGTGGAACTGCGTGACCCCGATTTCACGACCGCCGTGCGCGCTGCCGATACGATCAAT
GTCTTCGCCAAGCGCCGTTATGGTCGCGGGGTGGCCATTGCGCGCGATGCCAAGACGAT
CCGCCTGTCACGCCCGAAGAACGTGCCTGCCGCCCGTTTTCTTGCAGAGCTTGAGGGCC
TGCCGATCACCACCGACGAAGTGGCGCGGGTGGTAGTGGATGAGCGGACCGGAACGGTC
GTTATTGGCGAAAAGGTGCGCATTTCCAAGGTTGCGATCAGCCATGGCAGCCTGACGGT
CCGTGTGACGGAAACGCCGATGGTCGTCCAGCCAGAGCCGTTCAGTGACGGCGAGACGG
CGGTTGAGCCCAACACCGATATCGCCGTCAACCAGCAGAACGCCAAGATCGGCATCCTG
AGCGGCGCCAATCTCGAAAATCTCGTGAAGGGGCTCAATCAGATCGGCGTGAAGCCCAC
CGGCATCATCGCGATATTGCAGGCAATCAAGACATCCGGCGCGCTTCATGCCGAACTGG
TGGTGCAA

### Références

Akerley BJ, et al. Cell. 1995. *80*:611-20.
Anderson, T. D., and Cheville, N. F. (1986). Am. J. Pathol *124*, 226-237.
Angelichio, M. J., and Camilli, A. (2002). Infect. Immun. *70*, 6518-6523
Bellaire, B. H., et al. Infect. Immun. *67*, 2615-2618.
Bellaire, B. H., et al. Infect. Immun. *71*, 1794-1803 (2003b).
Boschiroli, M. L., Foulongne, V., and O'Callaghan, D. (2001). Curr. Opin. Microbiol. *4*, 58-64.
Cloeckaert, A., et al. Microbes Infect. *3*, 729-738 (2001).
Delrue, R. M. (2002). Contribution à l'analyse des mécanismes moléculaires impliqués dans le trafic intracellulaire de *Brucella melitensis* 16M (Namur, Presses universitaires de Namur).
DelVecchio, V. G., Kapatral, V., Redkar, R. J., Patra, G.,
Mujer, C., Los, T., Ivanova, N., Anderson, I., Bhattacharyya,
A., Lykidis, A., et al. (2002). Proc. Natl. Acad. Sci. U S A *99*, 443-448.
Edmons, M. D., et al. Am. J. Vet. Res. *62*, 1461-1466.
Essenberg, R. C., et al. Vet. Microbiol. *90*, 249-261.
Fernandez-Prada, C. M., et al. Infect. Immun. *71*, 2110-2119.
   Finlay, B. B., and Cossart, P. (1997). Science *276*, 718-725.
Finlay, B. B., and Falkow, S. (1997). Microbiol. Mol. Biol. Rev. *61*, 136-169.
Gross, A., et al. Infect. Immun. *68*, 342-351.
Guzman-Verri, C., et al. (2002). Proc. Natl. Acad. Sci. U S A *99*, 12375-12380.
Jubier-Maurin, V., et al.. Bacteriol. *183*, 426-434.
Ko, J., and Splitter, G. A. (2003). Clin. Microbiol. Rev. *16*, 65-78.
Kohler, S., et al. (2002). Proc. Natl. Acad. Sci. U S A *99*, 15711-15716.
Kohler, S., et al. (1999). Immun. *67*, 6695-6697.
Lopez-Goni, I., et al. (2002). Vet. Microbiol. *90*, 329-339.
Lopez-Goni, I., and Moriyon, I. (1995). Curr. Microbiol. *31*, 291-293.
Lopez-Goni, I., et al. (1992). Infect. Immun. *60*, 4496-4503.
Lundberg, B. E., et al. (1999). Immun. *67*, 436-438.
Mahan, M. J., et al. Annu. Rev. Genet. *34*, 139-164.
Moreno, E., et al. (2002). Vet. Microbiol. *90*, 209-227.
Parent, M. A., et al. (2002). Microb. Pathog. *32*, 239-248.
Porte, F., et al. (1999). Infect. Immun. *67*, 4041-4047.
Porte, F., et al. (2003). Infect. Immun. *71*, 1481-1490.
Sola-Landa, A., et al. Mol. Microbiol. *29*, 125-138.
Taminiau, B., et al. (2002). Infect. Immun. *70*, 3004-3011.
Tibor, A., et al. (2002). Infect. Immun. *70*, 5540-5546.
Ugalde RA. Microbes Infect. 1999. 1:1211-9. Review.

## Revendications

1. Séquence nucléotidique encodant une protéine flagellaire de *Brucella*.

2. Séquence nucléotidique selon la revendication 1, **caractérisée en ce qu'**elle présente plus de 80% de l'identité de séquence, de préférence plus de 90% de l'identité de séquence avec une séquence choisie parmi le groupe constitué parmi SEQID N°1 à SEQID N°50.

3. Séquence nucléotidique selon la revendication 2, **caractérisée en ce qu'**elle est choisie parmi le groupe constitué par la séquence SEQID N°1 à SEQID N°50.

4. Polypeptidique flagellaire de *Brucella* encodé par une séquence nucléotidique selon l'une quelconque des revendications précédentes 1 à 3.

5. Vecteur comprenant la séquence nucléotidique selon l'une quelconque des revendications précédentes 1 à 3 ou le polypeptide selon la revendication 4.

6. Cellule transformée ou comprenant le vecteur selon la revendication 5.

7. Inhibiteur dirigé contre la séquence nucléotidique selon l'une quelconque des revendications 1 à 4 ou contre le polypeptide selon la revendication 4.

8. Inhibiteur selon la revendication 7, **caractérisé en ce qu'**il est un anticorps (monoclonal ou polyclonal) ou une portion hypervariable de celui-ci.

9. Inhibiteur selon la revendication 7, **caractérisé en ce qu'**il est une séquence antisens ou un ribozyme susceptible de s'hybrider de manière complémentaire avec la séquence nucléotidique selon les revendications 1 à 3 pour inhiber son expression.

10. Cellule de Brucella dont une ou plusieurs des SEQID N°1 à SEQID N°50 ont été modifiées (par mutation et/ou délétion) de manière à perdre le caractère virulent de la cellule.

11. Composition pharmaceutique comprenant un véhicule ou un diluant pharmaceutique adéquat et un élément choisi parmi le groupe constitué par une séquence nuclétodique selon l'une quelconque des revendications 1 à 3, le polypeptide selon la revendication 4, le vecteur selon la revendication 5, la cellule selon la revendication 6 ou 10 ou l'inhibiteur selon l'une quelconque des revendications 7 à 9.

12. Composition pharmaceutique selon la revendication 11, **caractérisée en ce qu'**elle est une composition vaccinale, comprenant éventuellement un ou plusieurs adjuvants.

13. Utilisation de la composition selon la revendication 11 ou 12 pour la préparation d'un médicament destiné au traitement ou la prévention d'infections induites par *Brucella* chez un mammifère.

14. Trousse de diagnostic comprenant une séquence nucléotidique selon l'une quelconque des revendications 1 à 3, la séquence polypeptidique selon la revendication 4 et/ou un inhibiteur selon l'une quelconque des revendications précédentes 7 à 9.
